# EUROPÄISCHE PATENTSCHRIFT

(11) **EP 4 267 067 B1**
(45) Veröffentlichungstag und Bekanntmachung des Hinweises auf die Patenterteilung: **29.10.2025**
(21) Anmeldenummer: 21839226.4
(22) Anmeldetag: 21.12.2021
(51) Int. Cl.: A61F 13/84, A61F 13/532, A61F 13/534, A61F 13/537

(54) **INKONTINENZARTIKEL MIT KANAL UND PH-REGULATIONSMITTEL**
INCONTINENCE ARTICLE WITH CHANNEL AND PH REGULATOR
ARTICLE POUR INCONTINENCE AVEC RÉGULATEUR DE PH ET DE CANAL

(30) Priorität: 23.12.2020 DE 102020134878
(43) Veröffentlichungstag der Anmeldung: 01.11.2023
(73) Patentinhaber: Paul Hartmann AG, 89522 Heidenheim (DE)
(72) Erfinder: VECHTER, Olga, 89075 Ulm (DE); BÄHRLE, Christian, 89542 Herbrechtingen (DE); KESSELMEIER, Rüdiger, 89233 Burlafingen (DE); GAUSE, Enno, 89522 Heidenheim (DE)
(74) Vertreter: Paul Hartmann AG Patents & Licensing
(86) Internationale Anmeldenummer: PCT/EP2021/086973
(87) Internationale Veröffentlichungsnummer: WO 2022/136363

(56) Entgegenhaltungen:
- WO-A1-2019/167356
- WO-A1-2021/123085
- US-A1- 2011 224 637
- US-B2- 9 757 285

## Beschreibung

Die vorliegende Erfindung betrifft einen Inkontinenzartikel für die Aufnahme von Körperausscheidungen mit einem Mittel zur Regulation des pH-Wertes für die bevorzugte Verwendung durch Erwachsene.

Ein häufiges Problem bei der Verwendung von Inkontinenzartikeln ist das Auftreten von Hautreizungen oder Hautentzündungen, welche durch den Kontakt der Haut mit Körperausscheidungen wie beispielsweise Urin hervorgerufen werden. Oftmals kann sich eine sogenannte Inkontinenz-assoziierte Dermatitis (IAD) entwickeln, eine durch Kontakt mit einem Reizstoff wie Urin hervorgerufene Hautentzündung in der perinealen oder perigenitalen Region. Dabei spielen Veränderungen des pH-Wertes der Haut bei Kontakt mit dem alkalischeren Urin eine wesentliche Rolle. Um einen solchen Kontakt der Haut mit Urin zu verringern weisen Inkontinenzartikel typischerweise superabsorbierende Materialien auf, welche große Mengen an Urin aufnehmen und dauerhaft binden können. Außerdem kann mit einem entsprechenden Aufbau des Inkontinenzartikels, beispielsweise durch die Verwendung von Flüssigkeitsaufnahme- und Verteilerschichten im Inneren des Inkontinenzartikels, Flüssigkeiten schneller in den Inkontinenzartikel aufgenommen und dabei von der Haut des Benutzers weggeleitet werden. Teilweise verfügen Inkontinenzartikel auch über Öffnungen, Rillen oder Vertiefungen, welche zu einer besseren Flüssigkeitsleitung und -verteilung im Inkontinenzartikel beitragen sollen. Weiterhin werden manchmal Substanzen in das absorbierende Material des Saugkörpers eingebracht, um den pH-Wert der aufgenommenen Körperflüssigkeiten zu regulieren.

Jedoch kommt es häufig vor, dass bei einem Miktionsereignis eine große Menge an Urin in kurzer Zeit, also schwallartig, auf den Inkontinenzartikel trifft, so dass der Artikel diese Menge nicht schnell genug ableiten kann. Dabei kann sich der Urin zunächst im Schrittbereich des Inkontinenzartikels sammeln oder sich auf der Oberfläche des Saugkörpers vom Auftreffpunkt ausgehend in die Fläche ausbreiten, bevor er vom Saugkörper vollständig aufgesogen werden kann. Superabsorbierende Materialien können außerdem beim Aufquellen einer raschen Weiterleitung des Urins entgegenwirken. Dadurch kommt es zu einem längeren und großflächigen Hautkontakt, so dass leicht Hautreizungen entstehen können. Es besteht also weiterhin Bedarf an verbesserten Inkontinenzartikeln. Inkontinenzartikel für Erwachsene sind seit langem bekannt und weisen häufig ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper auf. Diese können grundsätzlich auf vielfältige Weise ausgestaltet sein, beispielsweise als direkt am Körper anliegende Inkontinenzprodukte oder als saugfähige Inkontinenzunterlagen.

Im Stand der Technik finden sich Ansätze, Inkontinenzartikel mit einem Mittel zur pH-Wert-Kontrolle bereitzustellen. WO2012121932A1 lehrt einen absorbierenden Artikel mit einer Pufferzusammensetzung, die mit absorbierendem Material vorgemischt ist oder die zwischen zwei Saugkörperschichten angeordnet ist. Dabei soll die Pufferzusammensetzung im trockenen Zustand jedenfalls keinen Kontakt zum Topsheet haben. WO2019167356 lehrt weitere absorbierende Artikel mit einem Mittel zu pH-Wert Kontrolle.

Die Erfinder haben erkannt, dass eine solche Anordnung den Nachteil hat, dass die Körperflüssigkeit, die beim Eindringen in den absorbierenden Kern in Kontakt mit der Pufferzusammensetzung kommt, diese beim Vordringen in tiefere Saugkörperlagen ausschwemmen kann oder überhaupt erst in tieferen Saugkörperlagen mit der Pufferzusammensetzung in Kontakt kommt. Außerdem kann bei einem Anfall einer großen Menge an Körperflüssigkeit diese häufig nicht schnell genug in den Saugkörper aufgesogen werden, so dass sich ein Teil der Flüssigkeit ohne Kontakt zu den die Pufferzusammensetzung enthaltenden Saugkörperlagen an der Oberfläche des Artikels ausbreiten kann. Die Kontrolle des pH-Wertes an einer hautseitigen Oberfläche des Artikels kann daher insbesondere bei schwallartigem Auftreffen von Körperflüssigkeiten auf den Inkontinenzartikel ungleichmäßig und/oder nur in ungenügendem Maße erfolgen. Superabsorbierende Materialien in körpernahen Lagen des Saugkörpers können diesen nachteiligen Effekt weiter verstärken, wenn durch sie eine Verzögerung der pH-Regulation hervorgerufen wird.

Der vorliegenden Erfindung liegt daher die Aufgabe zu Grunde, diese Nachteile zu überwinden.

Diese Aufgabe wird gelöst durch einen Inkontinenzartikel für die Aufnahme von Körperausscheidungen, umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper, wobei der Absorptionskörper eine erste Saugkörperlage umfasst, und wobei die erste Saugkörperlage mindestens einen in der Längsrichtung orientierten Kanal aufweist, wobei der Kanal eine sich durch die erste Saugkörperlage in Dickenrichtung hindurcherstreckende Ausnehmung umfasst, dadurch gekennzeichnet dass ein pH-Regulationsmittel in den Kanal eingebracht ist.

Da der Kanal der primären Aufnahme von Körperflüssigkeiten wie Urin und deren anschließenden Weiterleitung in weiter entfernte Regionen des Absorptionskörpers dient, unterstützt das im Kanal eingebrachte pH-Regulationsmittel eine rasche pH-Kontrolle, so dass der pH-Wert reguliert werden kann, bevor die Körperflüssigkeiten weitergeleitet und/oder bevor sie im Absorptionskörper dauerhaft gebunden werden.

Bevorzugt ist der Kanal parallel zu einer Längsmittelachse des Absorptionskörpers und/oder der ersten Saugkörperlage und/oder des Inkontinenzartikels angeordnet.

Insbesondere ist der Kanal auf einer Längsmittelachse des Inkontinenzartikels und/oder des Absorptionskörpers und/oder der ersten Saugkörperlage angeordnet.

Weiter insbesondere ist der Kanal symmetrisch zur Längsmittelachse des Inkontinenzartikels und/oder des Absorptionskörpers und/oder der ersten Saugkörperlage ausgebildet.

Der Kanal ist auf vorteilhafte Weise in Querrichtung des Absorptionskörpers und/oder der ersten Saugkörperlage zentriert angeordnet.

Vorteilhafterweise ist der Kanal in Längsrichtung des Inkontinenzartikels im Bereich des Auftreffens von Urin bei der Benutzung (Miktionsbereich) angeordnet.

Weiterhin erstreckt sich der Kanal in vorteilhafter Weise über eine Quermittelachse des Absorptionskörpers hinweg.

Ein bevorzugter Kanal ist derart angeordnet, dass er von einer hinteren, also im Benutzungszustand rückenseitig zu liegen kommenden Querkante des Absorptionskörpers weiter entfernt ist als von einer vorderen, also im Benutzungszustand bauchseitig zu liegen kommenden Querkante des Absorptionskörpers. Jedoch kann der Kanal auch gleich weit von der vorderen und hinteren Querkante des Absorptionskörpers entfernt oder weiter von der vorderen als von der hinteren Querkante des Absorptionskörpers entfernt angeordnet sein.

In einer bevorzugten Ausführungsform erstreckt sich der Kanal geradlinig in der Längsrichtung des Inkontinenzartikels und/oder des Absorptionskörpers und/oder der ersten Saugkörperlage. Dadurch kann die Flüssigkeit sich im Kanal ungehinderter in der Längsrichtung des Inkontinenzartikels ausbreiten.

Jedoch sind auch wellenförmige, gezackte oder bogenförmige oder jede andere Kanalform denkbar, die der Fachmann für sinnvoll erachtet.

Unterschiedliche Kanalformen wie beispielsweise rechteckig, mandelförmig, knochenförmig, T-förmig, dreieckig, oval, sternförmig oder verzweigt sind im Stand der Technik hinreichend bekannt.

In der Längsrichtung orientierter Kanal bedeutet im Rahmen dieser Erfindung, dass eine Erstreckung des Kanals in der Längsrichtung des Inkontinenzartikels und/oder des Absorptionskörpers größer ist als dessen Erstreckung in Querrichtung des Inkontinenzartikels und/oder des Absorptionskörpers. Die Erfinder haben festgestellt, dass für eine rasche Flüssigkeitsleitung in Längsrichtung gerade solche Kanäle vorteilhaft sind, deren Erstreckung in der Längsrichtung des Inkontinenzartikels und/oder des Absorptionskörpers größer ist als deren Erstreckung in Querrichtung des Inkontinenzartikels und/oder des Absorptionskörpers. Insbesondere rechteckige, mandelförmige oder ovale Kanäle haben sich als vorteilhaft erwiesen.

In einer bevorzugten Ausführungsform weist die erste Saugkörperlage einen ersten Bereich auf, wobei die erste Saugkörperlage in dem ersten Bereich superabsorbierendes Polymer (SAP) aufweist.

Der erste Bereich grenzt bevorzugt an den Kanal an, insbesondere umgibt der erste Bereich den Kanal vollständig. Daher kann eine im Benutzungszustand aus dem Kanal in die erste Saugkörperlage eintretende Körperflüssigkeit in allen Richtungen rasch dauerhaft absorbiert werden.

Der erfindungsgemäße Kanal umfasst eine sich durch die erste Saugkörperlage in Dickenrichtung hindurcherstreckende Ausnehmung. In einer bevorzugten Ausführungsform ist der Kanal aus einer sich durch die erste Saugkörperlage in Dickenrichtung hindurcherstreckenden Ausnehmung gebildet.

Dabei ist der Kanal bevorzugt im Wesentlichen frei von absorbierenden Materialien. Dazu kann der Kanal durch Weglassen des absorbierenden Materials oder durch Entfernen von absorbierendem Material mittels Schneiden oder Stanzen ausgebildet werden.

Ebenso denkbar ist jedoch, dass der Kanal durch Prägen oder Komprimieren der ersten Saugkörperlage ausgebildet wird.

Weiter bevorzugt ist der Kanal der ersten Saugkörperlage im Wesentlichen frei von SAP.

Das bietet den Vorteil, dass die in den Kanal aufgenommene Körperflüssigkeit nicht im Kanal vorzeitig dauerhaft gebunden wird, sondern ungehindert entlang des Kanals weitergeleitet und dadurch gleichmäßiger im Absorptionskörper verteilt werden kann. Insbesondere kann eine vorzeitige Bindung der aufgenommenen Körperflüssigkeit mittels SAP im Kanal die pH-Regulation der Körperflüssigkeit behindern oder zumindest verzögern. Dadurch kann also eine Gefahr des Verstopfens des Kanals durch aufquellendes SAP verringert werden.

Nach einer bevorzugten Ausführungsform weist die erste Saugkörperlage einen einzigen Kanal auf.

In einer alternativen Ausführungsform weist die erste Saugkörperlage mehr als einen Kanal, insbesondere 2-5, weiter insbesondere genau 2 Kanäle auf.

In einer bevorzugten Ausführungsform weist der Absorptionskörper eine zweite Saugkörperlage auf, wobei die zweite Saugkörperlage unterhalb der ersten Saugkörperlage, also zwischen der ersten Saugkörperlage und dem Backsheet angeordnet ist.

Weiter bevorzugt weist die zweite Saugkörperlage weniger als 20 Gewichtsprozent, 15 Gewichtsprozent, 10 Gewichtsprozent, 5 Gewichtsprozent SAP auf, insbesondere ist die zweite Saugkörperlage frei von SAP.

Die zweite Saugkörperlage bewirkt auf vorteilhafte Weise einen höheren Tragekomfort, insbesondere bei einem am Körper anliegenden Inkontinenzartikel, da von der Backsheetseite her ein weicherer haptischer Eindruck des Inkontinenzartikels erreicht wird. Hierzu enthält die zweite Saugkörperlage wenig oder kein SAP. Dem Fachmann ist außerdem bekannt, dass partikelförmiges Material abrasive Kräfte ausüben kann, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher bietet die zweite Saugkörperlage auch den Vorteil, dass die Gefahr der Beschädigung der Backsheet-Folie im Herstellungsprozess oder während der Benutzung des Inkontinenzartikels verringert wird.

Es hat sich als vorteilhaft erwiesen, wenn das pH-Regulationsmittel partikelförmig ausgebildet ist.

Bevorzugt weist mindestens 50 Gewichtsprozent des pH-Regulationsmittels eine Partikelgröße von 10 bis 2000 µm, insbesondere von 50 bis 1200 µm, weiter insbesondere von 80 bis 800 µm auf.

Weiter bevorzugt beträgt die flächenbezogene Menge an pH-Regulationsmittel in dem Kanal 5 bis 100 g/m², insbesondere 10 bis 50 g/m², weiter insbesondere 15 bis 30 g/m². Grundsätzlich sind auch pH-Regulationsmittel mit geringerer oder größerer Partikelgröße erhältlich und verwendbar.

Jedoch bieten die bevorzugten Partikelgrößen den Vorteil, dass sich Partikel einer solchen Größe bei Benetzung mit Körperausscheidungen wie einer Miktionsflüssigkeit langsamer auflösen als pH-Regulationsmittel geringerer Partikelgröße, so dass das pH-Regulationsmittel während der Benutzung nicht so schnell ausgewaschen wird und ein pHregulierender Effekt an einer der Haut des Benutzers zugewandten Oberseite des Inkontinenzartikels länger erhalten bleibt.

Zudem ist beispielsweise im Vergleich zu feinpudrigem pH-Regulationsmittel mit geringerer Partikelgröße die Gefahr des Migrierens während des Herstellungsprozesses und/oder im trockenen Zustand des Inkontinenzartikels und die Gefahr des Ausschwemmens in der Benutzungssituation verringert, so dass das pH-Regulationsmittel auch ohne zusätzliche Befestigungsmittel wie beispielsweise Klebstoff an einem Bestimmungsort, insbesondere im Kanal, verbleibt.

Zudem lässt sich ein pH-Regulationsmittel der bevorzugten Partikelgröße insbesondere in schnell laufenden Maschinen leichter dosieren und in den Inkontinenzartikel, insbesondere in den Kanal einarbeiten als beispielsweise feinpudrige pH-Regulationsmittel, da es im Herstellungsprozess zu weniger Staubentwicklung und damit verbundenem Materialverlust kommt.

Andererseits bieten Partikel dieser Größe auch den Vorteil, dass sie während der Benutzung des Inkontinenzartikels haptisch weniger wahrnehmbar sind als beispielsweise Granulate mit höherer Partikelgröße, wodurch der Tragekomfort des Inkontinenzartikels verbessert wird.

Körperausscheidungen wie Urin weisen typischerweise einen alkalischeren pH-Wert als die Haut auf und können deshalb zu Hautirritationen oder -entzündungen führen. Daher sind im Stand der Technik pH-Regulationsmittel für Inkontinenzartikel zur pH-Kontrolle von Körperausscheidungen beschrieben wie schwache Säuren oder Basen und deren jeweilige Salze oder Kombinationen davon, beispielsweise Carbonsäuren wie Citronensäure, Essigsäure, Äpfelsäure, Milchsäure und/oder deren jeweilige Salze wie beispielsweise Natriumsalze. Grundsätzlich sind alle Substanzen oder Zusammensetzungen denkbar, die eine geeignete Pufferwirkung aufweisen und bei denen eine geringe Gefahr für Hautirritationen besteht. Hierbei gilt im Rahmen der vorliegenden Erfindung, dass superabsorbierende Polymere (SAP), also insbesondere Polymer, die zumindest das 15-fache ihres Gewichts an 0,9 gewichtsprozentiger Kochsalzlösung absorbieren (gemessen nach NWSP 242.0.R2(15)), nicht als pH-Regulationsmittel zu verstehen sind.

Die Erfinder haben festgestellt, dass es diesbezüglich vorteilhaft ist, wenn das pH-Regulationsmittel Trinatriumcitrat, Mononatriumcitrat oder Dinatriumcitrat aufweist.

In einer bevorzugten Ausführungsform kann das pH-Regulationsmittel entweder Mononatriumcitrat und Trinatriumcitrat, oder Dinatriumcitrat und Trinatriumcitrat, oder Mononatriumcitrat und Dinatriumcitrat und Trinatriumcitrat aufweisen.

Durch die Auswahl oder Kombination der genannten Komponenten kann eine Feineinstellung der Pufferwirkung eines Inkontinenzartikels je nach angestrebter Benutzungsdauer oder Absorptionskapazität des Artikels erreicht werden, ohne dass wie im Falle der Verwendung von Citronensäure die Gefahr der Entstehung stark sauer reagierender Bereiche besteht.

Falls das pH-Regulationsmittel mehr als ein Citrat umfasst, weist das pH-Regulationsmittel vorzugsweise eine homogene Mischung der jeweiligen Citrate auf. Das hat den Vorteil, dass lokale Unterschiede in der Pufferwirkung des pH-Regulationsmittels vermieden werden können.

Die Begriffe Mononatriumcitrat, Dinatriumcitrat, und Trinatriumcitrat umfassen sowohl die nicht hydrierte Form als auch Hydrate der jeweiligen Substanz.

Bevorzugt besteht das pH-Regulationsmittel aus Mononatriumcitrat oder Dinatriumcitrat.

Ein Vorteil bei der Verwendung von Mononatriumcitrat ist beispielsweise, dass Mononatriumcitrat weniger hygroskopisch ist als beispielsweise Citronensäure und daher kann ein unerwünschter Eintrag von Feuchtigkeit in den Inkontinenzartikel vor der Benutzung verringert werden. Da Mononatriumcitrat weniger hygroskopisch ist als beispielsweise Citronensäure ergibt sich als weiterer Vorteil eine bessere Rieselfähigkeit, so dass sich Mononatriumcitrat leichter dosieren lässt.

In einer bevorzugten Ausführungsform besteht das pH-Regulationsmittel aus Mononatriumcitrat.

In einer weiteren bevorzugten Ausführungsform besteht das pH-Regulationsmittel aus Dinatriumcitrat.

Durch die Verwendung nur einer Komponente als pH-Regulationsmittel ergeben sich prozesstechnische Vorteile, da keine Vermischung verschiedener Komponenten oder Aufrechterhaltung einer homogenen Durchmischung während des Herstellungsprozesses erforderlich ist. Zudem wird eine gleichmäßige Pufferwirkung über die pH-Regulationsmittel enthaltenden Bereiche des Inkontinenzartikels hinweg weiter unterstützt.

Im Rahmen dieser Erfindung sind die Mengenangaben des pH-Regulationsmittels so zu verstehen, dass sie sich auf ein wasserfreies pH-Regulationsmittel, beispielsweise nicht hydriertes Mononatriumcitrat, beziehen. Falls das pH-Regulationsmittel ein oder mehrere Hydrate oder wie nachfolgend beschrieben mehr als 5%, insbesondere mehr als 10% additive Substanzen umfasst, wird die Gesamtmenge des pH-Regulationsmittels in g/m² derart angepasst, dass die Gesamtmenge an dem enthaltenen reinen und/oder nicht hydrierten pH-Regulationsmittel der vorstehenden bevorzugten Menge entspricht. Bevorzugt wird das Mononatriumcitrat oder Dinatriumcitrat oder Trinatriumcitrat im Wesentlichen in reiner Form verwendet, insbesondere mit einem Reinheitsgrad von mindestens 90%, weiter insbesondere mindestens 95%, weiter insbesondere mindestens 98%, weiter insbesondere mindestens 99%, weiter insbesondere 100%. Insbesondere wird das Mononatriumcitrat oder Dinatriumcitrat oder Trinatriumcitrat mit einer Reinheit verwendet, welche den Anforderungen der German Pharmaceutical Codex (DAC) oder der Commission Regulation (EU) 231/2012 genügt.

Dadurch können durch Rückstände anderer Substanzen ausgelöste Hautirritationen bei Benutzung des Inkontinenzartikels weitestgehend vermieden werden.

Es ist jedoch auch denkbar, dass das pH-Regulationsmittel eine geringe Menge einer oder mehrerer additiver Substanzen aufweist, beispielsweise Konfektionierungs- und/oder Konditionierungsmittel wie Trennmittel, Stabilisatoren, Staubbindemittel, Antibackmittel, Netzmittel, Koagulationsmittel, Antikoagulationsmittel, adhäsive oder oberflächenaktive Substanzen oder antimikrobielle Substanzen, Farb- oder Duftstoffe oder pH-Indikatoren. Solchenfalls beträgt der Anteil der einen oder mehreren anderen Substanzen an der Gesamtmenge des pH-Regulationsmittels insgesamt bevorzugt weniger als 10%, weiter bevorzugt weniger als 5%, weiter bevorzugt weniger als 2%, weiter bevorzugt weniger als 1%.

In einer bevorzugten Ausführungsform weist die erste Saugkörperlage in dem ersten Bereich zumindest eine Teilmenge des pH-Regulationsmittels auf.

Es hat sich als vorteilhaft erwiesen, auch außerhalb des Kanals pH-Regulationsmittel anzuordnen, so dass der pH-Wert der absorbierten Körperflüssigkeit auch über einen längeren Zeitraum im Inkontinenzartikel in einem nachfolgend näher beschriebenen hautfreundlichen Bereich gehalten werden kann. Außerdem kann hierdurch insbesondere bei großen Mengen an eindringender Körperflüssigkeit der pH-Wert effektiver reguliert werden. Zudem kann hierbei eine Körperflüssigkeit, insbesondere bei einem Auftreffen einer großen Flüssigkeitsmenge, die nicht sofort im Bereich des Auftreffens in den Absorptionskörper, dabei insbesondere in den Kanal, aufgenommen werden kann, in vorteilhafter Weise auch in dem umgebenden ersten Bereich der ersten Saugkörperlage pH-reguliert werden.

Partikelförmiges pH-Regulationsmittel kann mit dem absorbierenden Material der ersten Saugkörperlage gemischt sein, also im Wesentlichen innerhalb der ersten Saugkörperlage angeordnet sein.

Bevorzugt wird das pH-Regulationsmittel jedoch auf einer körperseitigen Oberfläche der ersten Saugkörperlage angeordnet. Dadurch kann eine auf den Inkontinenzartikel auftreffende Körperflüssigkeit mit dem pH-Regulationsmittel in Kontakt treten bevor sie in der Saugkörperlage weitergeleitet oder dauerhaft gebunden wird, wodurch die Wirkung des pH-Regulationsmittels beschleunigt werden kann.

Alternativ zu partikelförmigem pH-Regulationsmittel kann das pH-Regulationsmittel auch in gelöster Form, insbesondere als wässrige oder alkoholische Lösung in den Inkontinenzartikel eingebracht sein, insbesondere durch Besprühen oder Eintauchen und optional nachfolgendem Trocknen mindestens einer, insbesondere der ersten Saugkörperlage. Als weitere Alternative kann das pH-Regulationsmittel auch Suspension oder Paste in den Inkontinenzartikel eingebracht sein.

In einer bevorzugten Ausführungsform ist das pH-Regulationsmittel in der ersten Saugkörperlage eingebracht, wobei das pH-Regulationsmittel in der ersten Saugkörperlage ungleichförmig eingebracht ist, insbesondere derart, dass die flächenbezogene Menge des pH-Regulationsmittels in einem mittleren Längsabschnitt der Absorptionskörpers in g/m² größer ist als in einem vorderen und/oder einem hinteren Längsabschnitt des Absorptionskörpers.

Durch eine solche ungleichförmige Verteilung des pH-Regulationsmittels wird auf vorteilhafte Weise erreicht, dass das pH-Regulationsmittel vorrangig in einem Bereich des Auftreffens von Körperflüssigkeiten wie beispielsweise Urin auf den Inkontinenzartikel bereitgestellt ist, sodass auch beim Anfall großer Flüssigkeitsmengen eine effektive pH-Kontrolle ermöglicht wird.

Der mittlere Längsabschnitt des Absorptionskörpers ist hierbei in einem Schrittbereich des Inkontinenzartikels, mithin den Punkt oder Bereich des Auftreffens von Urin bei der Benutzung überfangend angeordnet und erstreckt sich über die Quermittelachse des Absorptionskörpers hinweg. Der vordere Längsabschnitt des Absorptionskörpers schließt sich in Längsrichtung unmittelbar an den mittleren Längsabschnitt in Richtung der vorderen Querkante des Absorptionskörpers an und erstreckt sich bis zu der vorderen Querkante des Absorptionskörpers.

Der hintere Längsabschnitt des Absorptionskörpers schließt sich in Längsrichtung unmittelbar an den mittleren Längsabschnitt in Richtung der hinteren Querkante des Absorptionskörpers an und erstreckt sich bis zu der hinteren Querkante des Absorptionskörpers. Die Grenzen zwischen mittlerem und vorderem Längsabschnitt und zwischen mittlerem und hinterem Längsabschnitt verlaufen jeweils geradlinig in Querrichtung des Inkontinenzartikels und orthogonal zu einer Längsmittelachse des Absorptionskörpers. Der mittlere Längsabschnitt des Absorptionskörpers ist symmetrisch zu der Quermittelachse des Absorptionskörpers angeordnet.

Eine Längserstreckung des mittleren Längsabschnitts des Absorptionskörpers beträgt 30-65 %, insbesondere 30-55 %, weiter insbesondere 35-50%, weiter insbesondere 40-45 %, weiter insbesondere 43 % einer Gesamtlängserstreckung des Absorptionskörpers.

Der mittlere, hintere und vordere Längsabschnitt des Absorptionskörpers erstreckt sich in Querrichtung des Absorptionskörpers bevorzugt über eine gesamte Breite des Absorptionskörpers, mithin von einer ersten Längskante bis zu einer zweiten Längskante. Als Längserstreckung ist zu verstehen eine maximale Längserstreckung eines Elementes oder Längsabschnitts.

Als Quererstreckung ist zu verstehen eine maximale Quererstreckung eines Elementes oder Längsabschnitts.

Der Absorptionskörper kann rechteckig, dreieckig, oval, T-förmig, sanduhrförmig, anatomisch, asymmetrisch oder in einer anderen dem Fachmann als geeignet erscheinenden Form ausgebildet sein.

Das Backsheet kann insbesondere ein atmungsaktives, jedoch im Wesentlichen flüssigkeitsdichtes Folienmaterial umfassen oder dadurch gebildet sein. Das Topsheet ist zumindest bereichsweise vorzugsweise von einem auf Vliesbasis beruhenden zumindest bereichsweise flüssigkeitsdurchlässigen Material gebildet.

Bevorzugt weisen der vordere und/oder der hintere Längsabschnitt des Absorptionskörpers pH-Regulationsmittel auf. Dadurch können auch Teilmengen von Urin rasch pH-reguliert werden, die sich bei einem schwallartigen Auftreffen gegebenenfalls zunächst an der Oberfläche des Inkontinenzartikels ausbreiten und die erst im hinteren oder vorderen Längsabschnitt des Absorptionskörpers aufgenommen werden.

Es ist auch denkbar, dass der vordere und/oder hintere Längsabschnitt des Absorptionskörpers im Wesentlichen frei von pH-Regulationsmittel sind.

In einer bevorzugten Ausführungsform ist 5,00-30,00 g/m², insbesondere 6,00-25,00 g/m², weiter insbesondere 7,00-20,00 g/m², weiter insbesondere 8,00-15,00 g/m² pH-Regulationsmittel in dem mittleren Längsabschnitt des Absorptionskörpers angeordnet und/oder in dem vorderen und/oder hinteren Längsabschnitt des Absorptionskörpers jeweils 0,00-10,00 g/m², insbesondere 0,10-9,00 g/m², weiter insbesondere 0,20-7,00 g/m², weiter insbesondere 0,30-5,00 g/m² pH-Regulationsmittel angeordnet
In einer bevorzugten Ausführungsform ist das pH-Regulationsmittel in der ersten Saugkörperlage derart eingebracht, dass die Konzentration des pH-Regulationsmittels in Gewichtsprozent in dem mittleren Längsabschnitt des Absorptionskörpers größer ist als in dem vorderen und/oder dem hinteren Längsabschnitt des Absorptionskörpers. Hierdurch kann auf vorteilhafte Weise die Menge an pH-Regulationsmittel in dem mittleren Längsabschnitt unabhängig von einer Erhöhung einer Gesamtmaterialmenge des Absorptionskörpers und damit einer Dicken- oder einer Dichteänderung im mittleren Längsabschnitt erhöht werden.

Hierbei ist zu erwähnen, dass der Absorptionskörper und/oder die erste Saugkörperlage eine gleichförmige Dicke und/oder eine gleichförmige Dichte aufweisen kann. Ebenso ist denkbar, dass der Absorptionskörper und/oder die erste Saugkörperlage ein Dicken- oder Höhenprofil oder ein Dichteprofil aufweist.

Das Einbringen des pH-Regulationsmittels in die erste Saugkörperlage kann auf unterschiedliche Arten erfolgen. Bevorzugt wird das pH-Regulationsmittel auf einer Oberfläche der ersten Saugkörperlage angeordnet, insbesondere auf einer dem Topsheet zugewandten Oberfläche der ersten Saugkörperlage. Denkbar ist jedoch auch, dass das pH-Regulationsmittel im Inneren der ersten Saugkörperlage, dabei entlang der Dickenrichtung der ersten Saugkörperlage gleichförmig oder ungleichförmig angeordnet wird.

Der Absorptionskörper des Inkontinenzartikels ist geeignet und dazu bestimmt, Körperausscheidungen, insbesondere Körperflüssigkeiten, insbesondere Urin aufzunehmen und dauerhaft zu speichern.

Dazu weist die erste Saugkörperlage bevorzugt superabsorbierendes Polymer (SAP) auf.

In einer vorteilhaften Ausführungsform ist das SAP in dem ersten Bereich der ersten Saugkörperlage derart eingebracht, dass die Konzentration des SAP in Gewichtsprozent in dem mittleren Längsabschnitt des ersten Bereichs der ersten Saugkörperlage gleich groß ist wie in dem vorderen und/oder dem hinteren Längsabschnitt des ersten Bereichs der ersten Saugkörperlage.

Hierdurch ergeben sich Vorteile bei der Herstellung, da beispielsweise das SAP mit dem weiteren Material des ersten Bereichs der ersten Saugkörperlage gleichförmig vorgemischt werden kann und eine schwierigere separate Dosierung und/oder Anordnung des SAP bei hoher Maschinengeschwindigkeit entfallen kann.

In einer bevorzugten Ausführungsform enthält der Absorptionskörper superabsorbierendes Polymermaterial (SAP) zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP kann beispielsweise partikelförmig oder faserförmig oder blatt- oder schaumförmig ausgebildet sein.

Das SAP kann weiterhin ein im Wesentlichen sortenreines Polymermaterial oder eine Mischung von zwei oder mehreren Polymermaterialien umfassen oder daraus bestehen.

Der Absorptionskörper kann weitere Materialien, wie Zellstofffasern ("fluff") oder Kunststofffasern enthalten. Denkbar ist weiterhin, die erste und/oder die zweite Saugkörperlage des Absorptionskörpers durch Anordnung von ein oder mehreren Schichten verschiedenen Materials, insbesondere aus Vliesmaterial auszubilden.

In einer weiteren bevorzugten Ausführungsform weist der Inkontinenzartikel mindestens eine Flüssigkeitsaufnahme- und Verteilerschicht (englisch: acquisition distribution layer, ADL) auf, welche zwischen dem Topsheet und der ersten Saugkörperlage angeordnet ist und wobei insbesondere die ADL den Kanal bereichsweise, insbesondere vollständig überfängt. Dadurch können Körperflüssigkeiten wie Urin rasch aufgenommen, verteilt und an die erste Saugkörperlage und/oder in den Kanal weitergeleitet werden.

Flüssigkeitsaufnahme- oder Verteilerschichten sind im Fachgebiet bereits bekannt und bestehen typischerweise aus einem Fasermaterial, insbesondere einem Vliesmaterial.

Die Fasern können natürlichen oder künstlichen Ursprungs sein und können von definierter Länge (Stapelfasern) oder kontinuierlich ("endlos") sein oder in-situ gebildet werden. Die Fasern können aus einem einzelnen Polymer oder einer Polymermischung (Einkomponentenfaser) oder aus mehr als einem einzelnen Polymer und/oder einer Polymermischung (Mehrkomponentenfaser) ausgebildet sein.

Eine Mehrkomponentenfaser weist einen Querschnitt auf, der mehr als einen einzelnen Abschnitt umfasst, wobei jeder dieser Abschnitte ein anderes Polymer oder eine andere Polymermischung umfasst. Der Begriff Mehrkomponentenfaser umfasst, ist aber nicht beschränkt auf, eine Zweikomponentenfaser. Die verschiedenen Komponenten von Mehrkomponentenfasern sind in im Wesentlichen unterschiedlichen Bereichen über den Querschnitt der Faser angeordnet und erstrecken sich kontinuierlich über die Länge der Faser. Eine Mehrkomponentenfaser kann einen Gesamtquerschnitt aufweisen, der Teilbereiche der zwei oder mehr unterschiedlichen Komponenten jeglicher Form oder Anordnung aufweist, einschließlich beispielsweise koaxialer Unterabschnitte, Kern- und Hüllenunterabschnitte, nebeneinander liegende Unterabschnitte, radiale Unterabschnitte, inselförmige Unterabschnitte, etc.

Eine Zweikomponentenfaser mit einer "Kern/Hüll-Struktur" hat einen Querschnitt, der Folgendes umfasst: zwei diskrete Abschnitte, von denen jeder aus einem Polymer oder einer Polymermischung besteht, worin das Hüllpolymer oder die Hüllpolymermischkomponente um das Kernpolymer oder die Kernpolymermischungskomponente herum angeordnet ist.

Das Flächengewicht von Vliesmaterialien wird in der Regel in Gramm pro Quadratmeter (g/m²) angegeben.

In einer bevorzugten Ausführungsform umfasst die Flüssigkeitsaufnahme- und Verteilerschicht Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisförmigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP.

Als eine Alternative ist es im Fachgebiet bekannt, ein Material aus chemisch modifizierten Zellulosefasern, beispielsweise quervernetzten Zellulosefasern, als ADL zu verwenden.

Die Flüssigkeitsaufnahme- und Verteilerschicht kann auch andere Materialien wie perforierte Folien oder Schäume oder dergleichen umfassen oder daraus bestehen.

Die Flüssigkeitsaufnahme- und Verteilerschicht kann den Absorptionskörper im Wesentlichen vollständig oder nur teilweise überfangen, mithin im Wesentlichen dieselbe flächenhafte Erstreckung oder eine zumindest bereichsweise geringere Erstreckung als die erste Saugkörperlage und/oder die zweite Saugkörperlage des Absorptionskörpers in Längs- und/oder Querrichtung des plan aufliegenden Inkontinenzartikels aufweisen. Bevorzugt überfängt die Flüssigkeitsaufnahme- und Verteilerschicht die erste Saugkörperlage und/oder die zweite Saugkörperlage des Absorptionskörpers zu 5-100%, insbesondere zu 10-90%, weiter insbesondere zu 15-80%, weiter insbesondere zu 15-70% ihrer flächenhaften Erstreckung.

Vorzugsweise ist die Flüssigkeitsaufnahme- und Verteilerschicht zumindest in einem Bereich angeordnet in welchem die Miktionsflüssigkeit in Gebrauch mit hoher Wahrscheinlichkeit auf den Inkontinenzartikel trifft (Miktionsbereich). Insbesondere erstreckt sich die Flüssigkeitsaufnahme- und Verteilerschicht über und im Bereich der Quermittelachse des Absorptionskörpers.

In einer bevorzugten Ausführungsform beträgt die flächenbezogene Menge an pH-Regulationsmittel in einem von der ADL überfangenen Bereich 5 bis 100 g/m², insbesondere 10 bis 50 g/m², weiter insbesondere 15 bis 30 g/m².

In einer bevorzugten Ausführungsform ist das pH-Regulationsmittel im Wesentlichen zwischen der Flüssigkeitsaufnahme- und Verteilerschicht und der ersten Saugkörperlage angeordnet.

Insbesondere sind zumindest 50 Gewichtsprozent, insbesondere mindestens 60 Gewichtsprozent, weiter insbesondere 70 Gewichtsprozent, weiter insbesondere zumindest 80 Gewichtsprozent des pH-Regulationsmittels zwischen der Flüssigkeitsaufnahme- und Verteilerschicht und der ersten Saugkörperlage angeordnet.

Daraus ergibt sich in vorteilhafter Weise, dass aufgenommene Körperflüssigkeiten bereits nach Passieren der Flüssigkeitsaufnahme- und Verteilerschicht mit dem pH-Regulationsmittel in Kontakt treten und der pH-Wert rasch reguliert werden kann.

Die Gesamtmenge an pH-Regulationsmittel pro Inkontinenzartikel, der insbesondere zur Benutzung durch Erwachsene vorgesehen ist, beträgt vorzugsweise 0,20 bis 3,00 g, weiter vorzugsweise 0,30 bis 2,50 g, weiter vorzugsweise 0,40 bis 2,00 g, weiter vorzugsweise 0,50 bis 1,50 g, weiter vorzugsweise 0,50 bis 1,00 g, weiter vorzugsweise 0,50 bis 0,80 g.

In einer bevorzugten Ausführungsform erhält der Inkontinenzartikel über mindestens zwei, insbesondere mindestens drei Miktionsereignisse hinweg, einen hautfreundlichen pH-Wert aufrecht.

Dadurch wird der Tragekomfort auch über einen längeren Benutzungszeitraum wie beispielsweise während der Nachtruhe verbessert.

Weiter bevorzugt beträgt der pH-Wert auf einer der Haut zugewandten Oberseite des Inkontinenzartikels einen Wert von 4,0-6,5, insbesondere 4,2-6,2, weiter insbesondere 4,3-6,0, weiter insbesondere 4,5-5,8, weiter insbesondere 4,7-5,7.

Bevorzugt weist die zweite Saugkörperlage weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels auf. Weiter insbesondere ist die zweite Saugkörperlage frei von pH-Regulationsmitteln.

Dadurch wird auf vorteilhafte Weise ein höherer Tragekomfort erreicht, insbesondere bei einem am Körper anliegenden Inkontinenzartikel, da von der Backsheetseite her ein weicherer haptischer Eindruck des Inkontinenzartikels erreicht wird. Wie vorgehend beschrieben kann partikelförmiges Material abrasive Kräfte ausüben, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher ist ein weiterer Vorteil, dass die Gefahr der Beschädigung der Backsheet-Folie im Herstellungsprozess oder während der Benutzung des Inkontinenzartikels verringert wird.

Bei dem Inkontinenzartikel kann es sich um unterschiedliche Ausführungsformen handeln wie beispielsweise Inkontinenzvorlagen, Inkontinenzeinlagen, Slipeinlagen, Inkontinenzwindeln offenen Typs, Inkontinenzwindeln geschlossenen Typs oder auch Krankenunterlagen.

Je nach Typ oder Ausführungsform kann der Inkontinenzartikel ein oder mehrere weitere im Stand der Technik hinlänglich bekannte Merkmale aufweisen wie beispielsweise Seitenteile, Verschlusssysteme, Flügel, Elastifizierungen, Auslaufschutzsysteme (z.B. Cuffs), Nässeanzeiger, Kennzeichnungsmittel, hautpflegende oder geruchsregulierende Substanzen oder Zusammensetzungen, Befestigungsmittel (z.B. Klebstoff, Haken) sowie andere dem Fachmann bekannte oder sinnvoll erscheinende Merkmale.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung und Beispielen. In der Zeichnung zeigt:
**Figur 1**
   schematische Darstellung in Draufsicht eines Inkontinenzartikels
**Figur 2a**
   schematisch, einen Querschnitt durch einen Inkontinenzartikel mit einer ersten Saugkörperlage, Kanal, ADL und pH-Regulationsmittel
**Figur 2b**
   schematisch, einen Querschnitt durch einen Inkontinenzartikel mit einer ersten Saugkörperlage mit SAP und einer zweiten Saugkörperlage, Kanal, ADL und pH-Regulationsmittel
**Figur 3**
   schematische Darstellung in Draufsicht einer Inkontinenzwindel offenen Typs mit Verschlusselementen

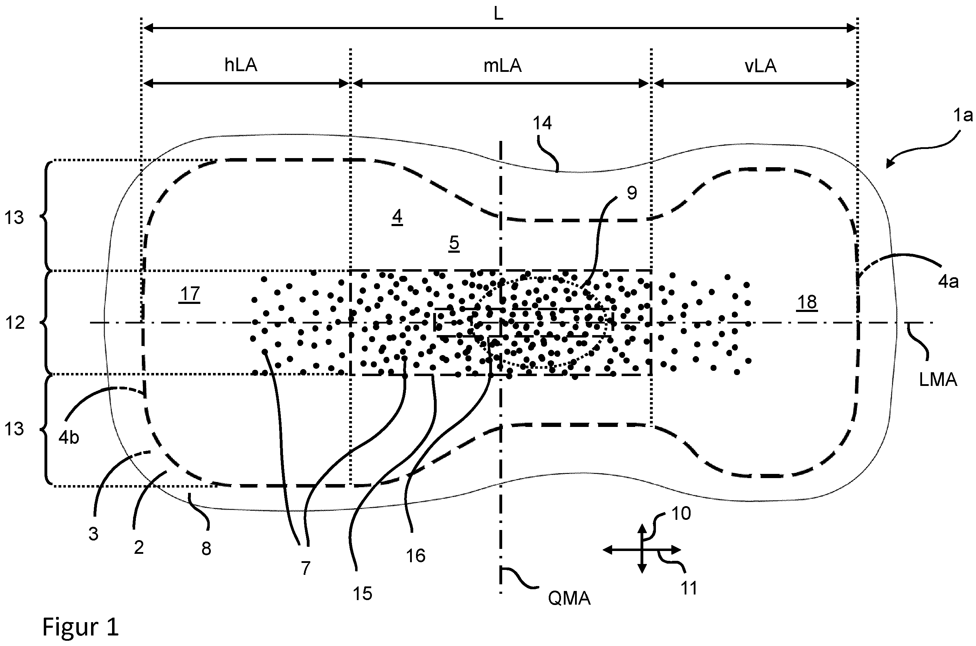

**Figur 1** zeigt schematisch, nicht maßstäblich, einen erfindungsgemäßen Inkontinenzartikel 1a mit einem zumindest bereichsweise flüssigkeitsdurchlässigen Topsheet 2, einem im Wesentlichen flüssigkeitsundurchlässigen Backsheet 3 und einen zwischen dem Topsheet 2 und dem Backsheet 3 angeordneten Absorptionskörper 4, wobei der Absorptionskörper 4 eine Quermittelachse QMA, eine Längsmittelachse LMA und eine erste Saugkörperlage 5 aufweist.

Die erste Saugkörperlage 5 weist einen in der Längsrichtung 11 orientierten Kanal 16 auf. Der Kanal 16 ist parallel zu und auf der Längsmittelachse LMA des Absorptionskörpers 4 und des Inkontinenzartikels 1a angeordnet. Weiter ist der Kanal 16 symmetrisch zur Längsmittelachse LMA des Absorptionskörpers 4 und Inkontinenzartikels 1a ausgebildet. Auf vorteilhafte Weise ist der Kanal 16 hier in Querrichtung 10 des Absorptionskörpers 4 zentriert angeordnet und erstreckt sich symmetrisch über die Quermittelachse QMA des Absorptionskörpers 4 hinweg.

Das Beispiel zeigt einen bevorzugten Kanal 16, der von einer hinteren, also im Benutzungszustand rückenseitig zu liegen kommenden Querkante 4b des Absorptionskörpers 4 weiter entfernt ist als von einer vorderen, also im Benutzungszustand bauchseitig zu liegen kommenden Querkante 4a des Absorptionskörpers 4. Jedoch kann der Kanal auch gleich weit von der vorderen und hinteren Querkante des Absorptionskörpers entfernt oder weiter von der vorderen als von der hinteren Querkante des Absorptionskörpers entfernt angeordnet sein.

Die vordere 4a und die hintere Querkante 4b des Absorptionskörpers 4 verlaufen hier bereichsweise gerade. Alternativ können die vordere 4a und/oder die hintere Querkante 4b des Absorptionskörpers auch vollständig gerade, abgewinkelt, gekrümmt, gebogen, wellenförmig oder mit einer anderen dem Fachmann als geeignet erscheinenden Linienführung verlaufen.

In diesem Beispiel ist der Kanal 16 rechteckig ausgebildet und erstreckt sich geradlinig in der Längsrichtung 11 des Absorptionskörpers und Inkontinenzartikels. Jedoch sind auch wellenförmige, gezackte oder bogenförmige oder jede andere Kanalform denkbar, die der Fachmann für sinnvoll erachtet. Unterschiedliche weitere Kanalformen wie beispielsweise mandelförmig, knochenförmig, T-förmig, dreieckig, oval, sternförmig oder verzweigt sind im Stand der Technik hinreichend bekannt.

Der Kanal 16 in diesem Beispiel weist auf vorteilhafte Weise eine größere Erstreckung in der Längsrichtung 11 des Absorptionskörpers 4 auf als in der Querrichtung 10.

Der Kanal 16 weist pH-Regulationsmittels 7, beispielsweise partikelförmiges Mononatriumcitrat oder Trinatriumcitrat oder Dinatriumcitrat, auf, hier punktförmig dargestellt. Dabei kann eine im Benutzungszustand in den Kanal 16 geleitete Körperflüssigkeit rasch auf das pH-Regulationsmittel 7 treffen.

Ein einzelner zentraler Kanal 16 wie hier in der **Figur 1** dargestellt wirkt sich vorteilhaft auf eine Stabilität und Form des Inkontinenzartikels 1a im Benutzungszustand aus. Jedoch ist auch denkbar, mehr als einen Kanal, insbesondere 2 Kanäle vorzusehen.

Das pH-Regulationsmittel 7 ist ungleichförmig in die erste Saugkörperlage 5 eingebracht. Dabei ist eine flächenbezogene Menge des pH-Regulationsmittels 7 in einem mittleren Längsabschnitt mLA des Absorptionskörpers 4 in g/m² größer als in einem vorderen vLA und/oder einem hinteren Längsabschnitt hLA des Absorptionskörpers 4 (in **Figur** 1 schematisch verdeutlicht durch eine höhere Anzahl und Dichte des punktförmig dargestellten pH-Regulationsmittels 7 im mittleren Längsabschnitt mLA).

Die Länge des mittleren Längsabschnitts mLA kann 30-65% einer Gesamtlängserstreckung L des Absorptionskörpers betragen. **Figur 1** veranschaulicht nur beispielhaft die Verortung eines mittleren Längsabschnitts mLA mit einer Länge von 43% der Gesamtlängserstreckung L des Absorptionskörpers. Sowohl ein beliebiger mittlerer mLA als auch ein beliebiger vorderer vLA und ein beliebiger hinterer Längsabschnitt hLA des Absorptionskörpers 4 weisen in diesem Beispiel pH-Regulationsmittel 7 auf.

Das pH-Regulationsmittel 7 ist in diesem Beispiel beidseitig der Längsmittelachse LMA des Absorptionskörpers 4, dabei in einem in Querrichtung 10 zentralen Bereich 12 des Absorptionskörpers 4 angeordnet, wobei in Querrichtung 10 periphere Bereiche 13 des Absorptionskörpers 4 frei von pH-Regulationsmittel 7 sind. Dadurch wird das pH-Regulationsmittel 7 dort fokussiert, wo Körperflüssigkeiten typischerweise vorrangig auf den Inkontinenzartikel 1a auftreffen, nämlich im zentralen Bereich 12. Das pH-Regulationsmittel 7 ist dabei auch in dem Miktionsbereich 9 (auch als Miktionspunkt bezeichnet) angeordnet, in welchem eine Miktionsflüssigkeit im Benutzungszustand mit hoher Wahrscheinlichkeit auf den Inkontinenzartikel 1a trifft. Es ist aber auch möglich, das pH-Regulationsmittel 7 sowohl im zentralen Bereich 12 als auch in den peripheren Bereichen 13 anzuordnen.

Der vordere vLA und der hintere Längsabschnitt hLA des Absorptionskörpers 4 weisen in dieser bevorzugten Ausführungsform in an die vordere 4a oder hintere Querkante 4b angrenzenden Teilabschnitten 17,18 kein pH-Regulationsmittel 7 auf. In einer alternativen Ausführungsform kann das pH-Regulationsmittel 7 jedoch über die gesamte Längserstreckung L des Absorptionskörpers 4 hinweg angeordnet sein. In einer weiteren alternativen Ausführungsform kann das pH-Regulationsmittel 7 nur innerhalb des mittleren Längsabschnitts mLA des Absorptionskörpers 4 angeordnet sein.

Der Absorptionskörper 4 und ein gemeinsam durch das Topsheet 2 und das Backsheet 3 gebildetes Chassis 8 sind in diesem Ausführungsbeispiel anatomisch ausgebildet. Sie können jedoch auch rechteckig, dreieckig, oval, T-förmig, sanduhrförmig, anatomisch, asymmetrisch oder in einer anderen dem Fachmann als geeignet erscheinenden Form ausgebildet sein.

Der mittlere Längsabschnitt mLA des Absorptionskörpers 4 ist in einem Schrittbereich 14 des Inkontinenzartikels 1a, mithin sich über den Miktionsbereich 9 erstreckend, angeordnet und erstreckt sich zudem über die Quermittelachse QMA des Absorptionskörpers 4 hinweg. Der mittlere Längsabschnitt mLA des Absorptionskörpers 4 ist dabei symmetrisch zu der Quermittelachse QMA des Absorptionskörpers 4 angeordnet.

Der vordere Längsabschnitt vLA des Absorptionskörpers 4 schließt sich in Längsrichtung 11 an den mittleren Längsabschnitt mLA in Richtung der vorderen Querkante 4a des Absorptionskörpers 4 an und erstreckt sich bis zu der vorderen Querkante 4a des Absorptionskörpers 4.

Der hintere Längsabschnitt hLA des Absorptionskörpers 4 schließt sich in Längsrichtung 11 an den mittleren Längsabschnitt mLA in Richtung der hinteren Querkante 4b des Absorptionskörpers 4 an und erstreckt sich bis zu der hinteren Querkante 4b des Absorptionskörpers 4. Die Grenzen zwischen mittlerem mLA und vorderem Längsabschnitt vLA und zwischen mittlerem mLA und hinterem Längsabschnitt hLA verlaufen jeweils geradlinig in Querrichtung 10 des Inkontinenzartikels 1a und orthogonal zu der Längsmittelachse LMA des Absorptionskörpers 4.

In diesem bevorzugten Beispiel entspricht die Längsmittelachse LMA des Absorptionskörpers 4 einer Längsmittelachse des Inkontinenzartikels 1a und einer Längsmittelachse der ersten Saugkörperlage 5. Ebenso entspricht hier die Längsrichtung 11 des Absorptionskörpers 4 einer Längsrichtung des Inkontinenzartikels 1a und einer Längsrichtung der ersten Saugkörperlage 5.

Weiterhin entspricht die Quermittelachse QMA des Absorptionskörpers 4 einer Quermittelachse des Inkontinenzartikels 1a und einer Quermittelachse der ersten Saugkörperlage 5. Ebenso entspricht hier die Querrichtung 10 des Absorptionskörpers 4 einer Querrichtung des Inkontinenzartikels 1a und einer Querrichtung der ersten Saugkörperlage 5.

Die erste Saugkörperlage 5 ist in diesem bevorzugten Ausführungsbeispiel deckungsgleich, also in einer Längsrichtung 11 des Absorptionskörpers 4 und in einer Querrichtung 10 des Absorptionskörpers 4 gleich weit erstreckt wie der Absorptionskörper 4. Daher entspricht die Gesamtlängserstreckung L des Absorptionskörpers einer Gesamtlängserstreckung der ersten Saugkörperlage 5 und eine maximale Quererstreckung der ersten Saugkörperlage 5 entspricht einer maximalen Quererstreckung des Absorptionskörpers 4. Alternativ können die erste Saugkörperlage 5 und der Absorptionskörper 4 in Querrichtung 10 oder Längsrichtung 11 des Absorptionskörpers unterschiedlich weit erstreckt sein.

In diesem Beispiel weist der Inkontinenzartikel 1a zusätzlich eine Flüssigkeitsaufnahme- und Verteilerschicht (ADL) 15 auf. Eine Erstreckung der ADL 15 in der Längsrichtung 11 des Absorptionskörpers 4 entspricht hier einer Längserstreckung des mittleren Längsabschnitts mLA. Der Kanal 16 ist vollständig von der ADL 15 überfangen.

Die ADL 15 überfängt in diesem Beispiel 18 % einer Gesamtfläche des Absorptionskörpers 4. Es wurde festgestellt, dass dieser Flächenanteil auf vorteilhafte Weise gleichzeitig eine effiziente Flüssigkeitsaufnahme und -Verteilung bietet und kostengünstig ist. Eine geeignete ADL 15 kann jedoch zwischen 5 und 100 % der Gesamtfläche des Absorptionskörpers 4 überfangen.

In diesem Ausführungsbeispiel ist die ADL 15 symmetrisch zur Quermittelachse QMA des Absorptionskörpers 4 angeordnet. Die ADL 15 kann jedoch auch asymmetrisch zur Quermittelachse QMA des Absorptionskörpers 4 oder zu einer Quermittelachse des Inkontinenzartikels angeordnet sein, mithin näher bei der vorderen Querkante 4a oder der hinteren Querkante 4b des Absorptionskörpers als bei der jeweiligen anderen hinteren 4b oder vorderen Querkante 4a des Absorptionskörpers 4 angeordnet sein.

Sowohl die ADL 15 als auch der Kanal 16 erstrecken sich in Längsrichtung 11 über den Miktionsbereich 9.

Der in Figur 1 dargestellte Inkontinenzartikel kann zusätzliche optionale Merkmale aufweisen, die im Stand der Technik hinreichend beschrieben sind, wie beispielsweise Elastifizierungsmittel, Flüssigkeitsbarrieren (z.B. Cuffs) oder Befestigungsmittel.

Die **Figur 2a** zeigt in beispielhafter Weise schematisch, nicht maßstäblich, einen Querschnitt entlang der Quermittelachse QMA eines Inkontinenzartikels. Der Inkontinenzartikel 1b weist die in **Figur 1** dargestellten Merkmale auf.

Der Inkontinenzartikel 1b weist ein flüssigkeitsdurchlässiges Topsheet 2, ein im Wesentlichen (mithin in Gebrauch) flüssigkeitsundurchlässiges Backsheet 3, und einen dazwischen angeordneten Absorptionskörper 4 auf. Der Absorptionskörper 4 des Inkontinenzartikels 1b weist eine erste Saugkörperlage 5 mit Fasermaterial 19, wie beispielsweise Zellstofffasern oder Kunststofffasern, und partikelförmiges SAP 20 auf. Das SAP 20 ist im gezeigten Fall gleichförmig verteilt in der ersten Saugkörperlage 5 angeordnet. Die ADL 15 ist zwischen der ersten Saugkörperlage 5 und dem Topsheet 2 angeordnet.

In dieser bevorzugten Ausführungsform ist das pH-Regulationsmittel 7 zwischen der ersten Saugkörperlage 5 und der ADL 15 angeordnet, mithin auf einer dem Topsheet 2 zugewandten Oberfläche der ersten Saugkörperlage 5. Es ist vorteilhaft, wenn von der Topsheetseite eindringende Körperflüssigkeiten zunächst auf das pH-Regulierungsmittel 7 treffen bevor sie durch SAP 20 dauerhaft gebunden werden, da so eine effektivere pH-Wert-Kontrolle erfolgen kann.

Alternativ kann das pH-Regulationsmittel 7 auch im Inneren der ersten Saugkörperlage 5, dabei entlang der Dickenrichtung 21 der ersten Saugkörperlage 5 gleichförmig oder ungleichförmig angeordnet sein.

Der Inkontinenzartikel 1b weist außerdem einen Kanal 16 auf. Der Kanal 16 erstreckt sich in diesem Beispiel nicht vollständig durch die erste Saugkörperlage 5 hindurch.

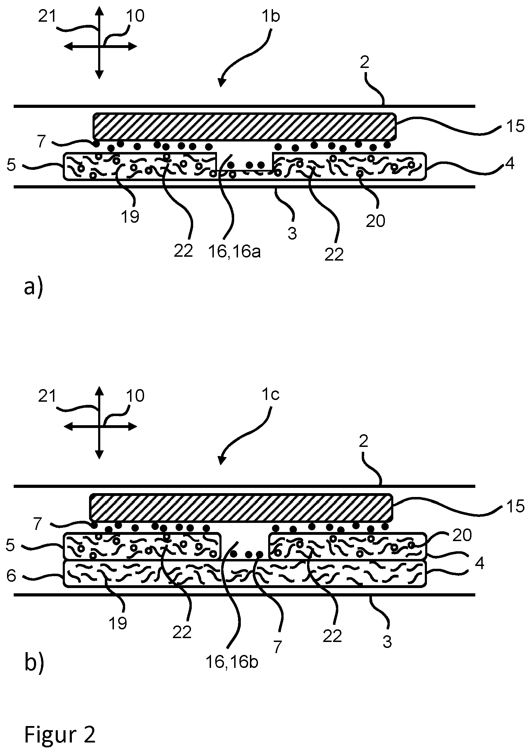

Dabei ist der Kanal 16 im Wesentlichen frei von absorbierenden Materialien, wie SAP 20 und Fasermaterial 19. Der Kanal 16 kann alternativ, wie nachfolgend in Bezug zu **Figur 2b** näher beschrieben, als eine sich durch die erste Saugkörperlage 5 in Dickenrichtung 21 vollständig hindurcherstreckende Ausnehmung gebildet sein.

In der in **Figur 2a** dargestellten Ausführungsform weist die erste Saugkörperlage 5 einen einzigen Kanal 16 auf, der auf vorteilhafte Weise in Querrichtung 10 des Absorptionskörpers 4 zentriert angeordnet ist.

Alternativ kann die erste Saugkörperlage mehr als einen Kanal, insbesondere 2-5, weiter insbesondere genau 2 Kanäle aufweisen.

Die erste Saugkörperlage 5 weist einen ersten Bereich 22 auf, der an den Kanal 16 angrenzt und der die gesamte Menge des SAP 20 der ersten Saugkörperlage 5 enthält.

Die **Figur 2b** zeigt schematisch, nicht maßstäblich, einen Querschnitt entlang der Quermittelachse QMA eines weiteren bevorzugten Inkontinenzartikels 1c.

Der Inkontinenzartikel 1c weist wie mit Bezug zu **Figur 2a** näher beschrieben ein flüssigkeitsdurchlässiges Topsheet 2, ein im Wesentlichen (mithin in Gebrauch) flüssigkeitsundurchlässiges Backsheet 3, und einen dazwischen angeordneten Absorptionskörper 4 auf sowie eine Flüssigkeitsaufnahme- und Verteilerschicht (ADL) 15 auf. Zusätzlich weist der Inkontinenzartikel 1c in dieser bevorzugten Ausführungsform noch eine zweite Saugkörperlage 6 auf. Die zweite Saugkörperlage 6 ist unterhalb der ersten Saugkörperlage 5 angeordnet, also zwischen erster Saugkörperlage 5 und dem Backsheet 3. In dieser bevorzugten Ausführungsform weist die zweite Saugkörperlage 6 kein SAP auf. Weiterhin weist die zweite Saugkörperlage 6 auch kein pH-Regulierungsmittel 7 auf. Dadurch wird der Tragekomfort des Inkontinenzartikels von der Backsheetseite verbessert und die Gefahr von Beschädigungen des Backsheets durch Partikel verringert. Alternativ können geringe Mengen an SAP und/oder pH-Regulierungsmittel in der zweiten Saugkörperlage 6 vorgesehen sein.

Die zweite Saugkörperlage 6 besteht im Wesentlichen aus Fasermaterial 19, wie beispielsweise Zellstofffasern oder Kunststofffasern.

Der Inkontinenzartikel 1c weist weiter einen Kanal 16 auf, in dem pH-Regulationsmittel 7 angeordnet ist. Der Kanal 16 ist in diesem Ausführungsbeispiel als eine sich durch die erste Saugkörperlage 5 in Dickenrichtung 21 vollständig hindurcherstreckende Ausnehmung 16b gebildet. Der Kanal 16 kann sich ebenso wie mit Bezug zu **Figur 2a** näher beschrieben nicht vollständig durch die erste Saugkörperlage 5 hindurch erstrecken.

In den in **Figuren 1** **und** **2a,b** dargestellten Ausführungsbeispielen umfasst das pH-Regulationsmittel 7 partikelförmiges Mononatriumcitrat, Typ "Fine Granular F3500" von Jungbunzlauer. Dieses Mononatriumcitrat weist zu 59 Gewichtsprozent eine Partikelgröße von 2 bis 355 µm und eine Reinheit von mindestens 99 % auf.

In den in **Figuren 1** **und** **2a,b** dargestellten Ausführungsbeispielen ist das SAP 20 partikelförmig ausgebildet. In einer denkbaren Variante kann das SAP faserförmig oder blatt- oder schaumförmig ausgebildet sein.

Die ADL 15 überfängt die erste Saugkörperlage 5 des Absorptionskörpers 4 nur teilweise und weist vorliegend eine geringere Erstreckung als die erste Saugkörperlage 5 (**Figuren 2a****,b**) und gegebenenfalls die zweite Saugkörperlage 6 (**Figur 2b**) auf.

Alternativ kann die die ADL 15 die erste Saugkörperlage 5 und/oder die zweite Saugkörperlage 6 vollständig überfangen.

Die erste Saugkörperlage 5 ist in diesem Fall (**Figur 2b**) in der Querrichtung 10 im Wesentlichen gleich erstreckt wie die zweite Saugkörperlage 6 des Absorptionskörpers 4. Die erste Saugkörperlage 5 und die zweite Saugkörperlage 6 können aber auch unterschiedlich erstreckt sein.

Der erste Bereich 22 der ersten Saugkörperlage 5 und die zweite Saugkörperlage 6 sind in den beschriebenen bevorzugten Ausführungsbeispielen als gleichförmig dicke Lagen, also mit einer gleichförmigen Erstreckung in der Dickenrichtung 21, dargestellt. Gleichwohl kann der erste Bereich 22 der ersten Saugkörperlage 5 und/oder die zweite Saugkörperlage 6 ein Dickenprofil aufweisen.

Die in **Figuren 2a,b** dargestellten Ausführungsformen sind so zu verstehen, dass sie ein oder mehrere weitere typische Merkmale von Inkontinenzartikeln aufweisen können, insbesondere dass sie als verschiedene Typen von Inkontinenzartikeln ausgebildet sein können wie beispielsweise Inkontinenzvorlagen, Inkontinenzeinlagen, Slipeinlagen, Windeln des offenen oder des geschlossenen Typs oder Krankenunterlagen.

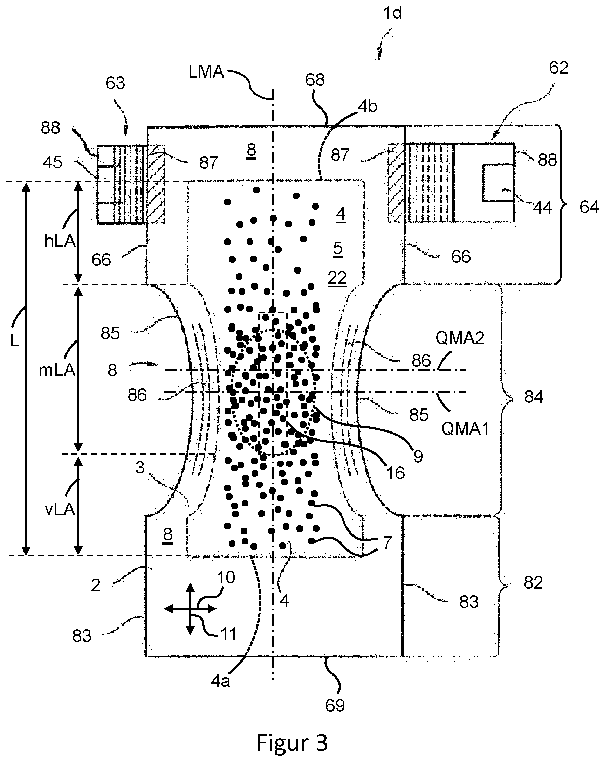

Die **Figur 3** zeigt, nicht maßstäblich, sondern schematisch einen insgesamt mit dem Bezugszeichen 1d bezeichneten erfindungsgemäßen Inkontinenzartikel, beispielhaft eine Inkontinenzwindel offenen Typs mit Verschlusselementen in der sogenannten T-Form für Erwachsene. Der Inkontinenzartikel 1d umfasst ein gemeinsam durch das Topsheet 2 und das Backsheet 3 gebildetes Chassis 8 mit einem Körperflüssigkeiten absorbierenden Absorptionskörper 4. Der Absorptionskörper 4 umfasst in diesem Ausführungsbeispiel eine erste Saugkörperlage 5, die SAP 20 (in **Figur 3** nicht gezeigt) enthält.

Weiter weist der Inkontinenzartikel 1d einen in der Längsrichtung orientierten Kanal 16 auf. Der Kanal 16 ist auf der Längsmittelachse LMA des Inkontinenzartikels 1d angeordnet und weist, wie mit Bezug zu **Figuren 1** **und** **2a,b** näher beschrieben, pH-Regulationsmittel 7 auf. Der Kanal 16 ist von dem ersten Bereich 22 der ersten Saugkörperlage 5 vollständig umgeben, wobei der erste Bereich 22 die gesamte Menge des SAP 20 der ersten Saugkörperlage 5 aufweist und der Kanal 16 im Wesentlichen frei von SAP 20 ist Alternativ kann der Inkontinenzartikel auch mehr als einen Kanal, insbesondere zwei Kanäle aufweisen.

In diesem Ausführungsbeispiel ist der Kanal 16 rechteckig ausgebildet. Es ist jedoch auch denkbar, dass der Kanal mandelförmig, knochenförmig, T-förmig, dreieckig, oval, sternförmig oder verzweigt ausgebildet ist.

Der Kanal 16 ist in diesem Beispiel derart angeordnet, dass er von der hinteren, also im Benutzungszustand rückenseitig zu liegen kommenden Querkante 4b des Absorptionskörpers 4 weiter entfernt ist als von der vorderen, also im Benutzungszustand bauchseitig zu liegen kommenden Querkante 4a des Absorptionskörpers 4. Jedoch kann der Kanal auch gleich weit von der vorderen und hinteren Querkante des Absorptionskörpers entfernt oder weiter von der vorderen als von der hinteren Querkante des Absorptionskörpers entfernt angeordnet sein.

Weiter ist der Kanal 16 hier in Querrichtung 10 des Absorptionskörpers 4 und der ersten Saugkörperlage 5 zentriert angeordnet und erstreckt sich in Längsrichtung 11 des Inkontinenzartikels 1d über den Miktionsbereich 9 hinweg.

Weiterhin erstreckt sich der Kanal 16 über die Quermittelachse QMA2 des Absorptionskörpers 4 hinweg.

Das SAP 20 ist dabei gleichförmig verteilt in der ersten Saugkörperlage 5 angeordnet, wie mit Bezug zu **Figur 2a** bereits ausgeführt.

Optional kann der Inkontinenzartikel auch eine wie mit Bezug zu **Figur 2b** näher beschriebene und dort mit Bezugszeichen 6 versehene zweite Saugkörperlage, die frei von pH-Regulationsmittel ist, und eine wie mit Bezug zu **Figuren 1** **und** **2a,b** näher beschriebene und dort mit Bezugszeichen 15 bezeichnete ADL umfassen.

Der Inkontinenzartikel 1d enthält ein pH-Regulationsmittel 7, bevorzugt umfassend Mononatriumcitrat oder Trinatriumcitrat oder Dinatriumcitrat oder bestehend aus Mononatriumcitrat oder Dinatriumcitrat, das auf einer dem Topsheet 2 zugewandten Oberseite der ersten Saugkörperlage 5 des Absorptionskörpers 4 angeordnet ist. In diesem Ausführungsbeispiel ist das pH-Regulationsmittel 7 im vorderen vLA, mittleren mLA und hinteren Längsabschnitt hLA des Absorptionskörpers 4 angeordnet, dabei über die gesamte Längserstreckung L des Absorptionskörpers 4 hinweg und dabei auch im Miktionsbereich 9. Der mittlere mLA, vordere, vLA und hintere Längsabschnitt hLA des Absorptionskörpers 4 entsprechen in ihrer planen Ausdehnung entlang der Längsrichtung 11 und Querrichtung 10 des Absorptionskörpers 4 in diesem Beispiel jeweils einem mittleren, vorderen und hinteren Längsabschnitt des ersten Bereichs 22 der ersten Saugkörperlage 5.

Die Längserstreckung des mittleren Längsabschnitts mLA des Absorptionskörpers 4 beträgt in diesem Beispiel 62 % der Gesamtlängserstreckung L des Absorptionskörpers 4.

In diesem Ausführungsbeispiel entspricht eine Quermittelachse QMA1 des Inkontinenzartikels 1d nicht einer Quermittelachse QMA2 des Absorptionskörpers 4. Der Absorptionskörper 4 und folglich auch die Quermittelachse QMA2 des Absorptionskörpers 4 sind näher an einer hinteren Querkante 68 des Inkontinenzartikels 1d angeordnet als an einer vorderen Querkante 69 des Inkontinenzartikels 1d. Alternativ kann der Absorptionskörper 4 und folglich auch die Quermittelachse QMA2 des Absorptionskörpers 4 gleich weit zur vorderen 69 und zur hinteren Querkante 68 oder näher an der vorderen 69 als an der hinteren Querkante 69 des Inkontinenzartikels 1g angeordnet sein.

Das pH-Regulationsmittel 7 ist ungleichförmig in die erste Saugkörperlage 5 eingebracht derart, dass im gezeigten Fall die Konzentration und die flächenbezogene Menge an pH-Regulationsmittel 7 in g/m² größer ist im mittleren Längsabschnitt mLA als im vorderen vLA und im hinteren Längsabschnitt hLA des Absorptionskörpers 4.

Bei dem Inkontinenzartikel 1d ist eine Längsrichtung 11 und eine Querrichtung 10 des Inkontinenzartikels 1d, des Chassis 8, des Absorptionskörpers 4 und der ersten Saugkörperlage 5 unterscheidbar, wobei die Querrichtung 10 im angelegten Zustand des Inkontinenzartikels 1d in einem Vorderbereich 82 und einem Rückenbereich 64 des Chassis 8 einer Hüftumfangsrichtung des Benutzers entspricht. Zwischen dem Vorderbereich 82 und dem Rückenbereich 62 ist ein Schrittbereich 84 angeordnet.

An einem jeweiligen Längsrand 85 des Schrittbereichs 84 angrenzend weist das Chassis 8 je einen elastifizierten Abschnitt, mithin einen elastifizierten Beinöffnungsabschnitt 86 auf. Diese elastifizierten Beinöffnungsabschnitte 86 sind im dargestellten Fall gebildet durch zwischen Topsheet 2 und Backsheet 3 verlaufende und in vorgespanntem Zustand an Topsheet 2 und/oder Backsheet 3 fixierte elastische Fäden, die bogenförmig gekrümmt, mithin zumindest mit einer Komponente in Längsrichtung 11 des Inkontinenzartikels 1d orientiert sind.

Der Vorderbereich 82 weist vordere seitliche Längsränder 83 auf, und der Rückenbereich 64 weist hintere seitliche Längsränder 66 auf.

Bei diesem als T-förmige Inkontinenzwindel ausgebildeten Inkontinenzartikel 1d ist im Rückenbereich 64 in Querrichtung 10 beidseitig ein seitlich über den jeweiligen hinteren seitlichen Längsrand 66 hinaus erstrecktes elastisches Windelseitenteil 62,63 vorgesehen, das im Bereich des jeweiligen hinteren seitlichen Längsrandes 66 in einem Überlappungsbereich 87 unlösbar an den Rückenbereich 64 des Chassis 8 angefügt ist. Im Vorderbereich 82 sind hingegen keine Windelseitenteile vorgesehen.

Die elastischen Windelseitenteile 62,63 der als T-förmige Inkontinenzwindel ausgebildeten Inkontinenzartikels 1d haben im Bereich ihres in Querrichtung 10 freien Endes 88 jeweils wenigstens ein Verschlusselement 44,45. Das Verschlusselement 44,45 ist in Form einer vorzugsweise rechteckigen Lasche ausgebildet und herstellerseitig auf sich selbst eingefaltet. In der Gebrauchssituation lässt sich das Verschlusselement 44,45 öffnen, das heißt wieder auffalten, um den Inkontinenzartikel 1d an einen Benutzer anzulegen, wobei die elastischen Windelseitenteile 62,63 in Überlappung mit dem Vorderbereich 82 des Chassis 8 gebracht werden und die Verschlusselemente 44,45 lösbar haftend auf einer vom Benutzer abgewandten Seite des Vorderbereichs 82 des Chassis 8 festgelegt werden. Das elastische Windelseitenteil 62 ist in der **Figur 3** in einem ungefalteten Zustand und das elastische Windelseitenteil 63 in einem zweifach auf sich selbst gefalteten Zustand dargestellt.

In einer alternativen Ausführungsvariante kann der Inkontinenzartikel auch als H-förmige Inkontinenzwindel ausgebildet sein, wobei dann zusätzlich im Vorderbereich und bevorzugt beidseitig Windelseitenteile ausgebildet sind, wie das beispielsweise in WO2005102241A1 gezeigt ist. Als weitere Ausführungsvariante ist auch eine Inkontinenzwindel des geschlossenen Typs denkbar, wobei solchen Falls vorzugsweise im Vorderbereich und im Rückenbereich jeweils ein Bauch- bzw. Rückenteil angefügt ist, welche an jeweiligen seitlichen Längsrändern des Bauch- und Rückenteils miteinander gefügt sein derart, dass die Inkontinenzwindel in Hüftumfangsrichtung ringförmig geschlossen ist, wie beispielsweise in WO2013171068A1 gezeigt. Als weitere Ausführungsvariante kann der Inkontinenzartikel als Krankenunterlage oder Inkontinenzvorlage ausgebildet sein.

### Ausführungsbeispiel 1: ungleichförmige Verteilung von pH-Regulationsmittel

In einen ersten 1A und einen zweiten erfindungsgemäßen Inkontinenzartikel 1B für Erwachsene wurde pH-Regulationsmittel eingebracht, wobei das pH-Regulationsmittel aus Mononatriumcitrat besteht. Bei dem ersten und zweiten Inkontinenzartikel handelt es sich um eine Inkontinenzvorlage mit einem flüssigkeitsdurchlässigen Topsheet, einem im Wesentlichen flüssigkeitsundurchlässigen Backsheet und einem dazwischen angeordneten Absorptionskörper mit einer ersten Saugkörperlage.

Zusätzlich weisen der erste und der zweite Inkontinenzartikel in diesem Beispiel eine zwischen der ersten Saugkörperlage und dem Backsheet angeordnete zweite Saugkörperlage und eine zwischen Topsheet und erster Saugkörperlage angeordnete ADL auf, wie in **Figur 2b** gezeigt). Der erste 1A und zweite Inkontinenzartikel 1B ist jeweils anatomisch geformt, wie in **Figur 1** dargestellt.

Die erste Saugkörperlage weist außerdem jeweils einen in Längsrichtung des Inkontinenzartikels orientierten und auf einer Längsmittelachse des Inkontinenzartikels angeordneten Kanal und einen den Kanal vollständig umgebenden ersten Bereich mit SAP auf. Die Konzentration des SAP ist im mittleren und vorderen und hinteren Längsabschnitt des ersten Bereichs der ersten Saugkörperlage gleich groß.

Der Kanal ist als eine sich in der Dickenrichtung hindurcherstreckende Ausnehmung der ersten Saugkörperlage gebildet und im Wesentlichen frei von SAP.

Die ADL ist im mittleren Längsabschnitt des Absorptionskörpers angeordnet derart, dass sie den Kanal vollständig überfängt.

Die zweite Saugkörperlage ist frei von SAP und frei von Mononatriumcitrat ausgebildet.

Das Mononatriumcitrat ist ungleichförmig in die erste Saugkörperlage eingebracht, dabei auf einer dem Topsheet zugewandten Oberfläche der ersten Saugkörperlageangeordnet. Das Mononatriumcitrat ist folglich (in der Dickenrichtung betrachtet) zwischen der ersten Saugkörperlage und dem Topsheet und - innerhalb des von der ADL überfangenen Bereiches der ersten Saugkörperlage - zwischen der ersten Saugkörperlage und der ADL angeordnet. Eine Teilmenge des Mononatriumcitrats ist im Kanal angeordnet.

Die flächenbezogene Menge des Mononatriumcitrats ist in dem mittleren Längsabschnitt des Absorptionskörpers in g/m² größer als im vorderen und hinteren Längsabschnitt (**Tabelle 1**). Der erste Inkontinenzartikel 1A weist 0,60 g Mononatriumcitrat und der zweite Inkontinenzartikel 1B weist 0,7 g Mononatriumcitrat auf (**Tabelle 1**). Dabei ist das Mononatriumcitrat im ersten Inkontinenzartikel 1A eingebracht derart, dass 67,9 ± 2,8 Gewichtsprozent des Mononatriumcitrats im mittleren Längsabschnitt des Absorptionskörpers und 10,0 ± 1,7 Gewichtsprozent im vorderen Längsabschnitt und 22,1 ± 2,2 Gewichtsprozent im hinteren Längsabschnitt des Absorptionskörpers angeordnet ist. Im zweiten Inkontinenzartikel 1A ist das Mononatriumcitrat eingebracht derart, dass 91,9 ± 2,7 Gewichtsprozent des Mononatriumcitrats im mittleren Längsabschnitt des Absorptionskörpers und 1,8 ± 0,2 Gewichtsprozent im vorderen Längsabschnitt und 6,3 ± 1,3 Gewichtsprozent im hinteren Längsabschnitt des Absorptionskörpers angeordnet ist.

Als pH-Regulationsmittel wurde partikelförmiges Mononatriumcitrat, Typ "Fine Granular F3500" von Jungbunzlauer verwendet. Dieses Mononatriumcitrat weist zu 59 Gewichtsprozent eine Partikelgröße von 2 bis 355 µm und eine Reinheit von mindestens 99 % auf

**Tabelle 1: Menge und Verteilung des pH-Regulationsmittels (Mononatriumcitrat) im ersten 1A und zweiten Inkontinenzartikel 1B, angegeben sind Mittelwerte und Standardabweichungen aus n=6**

| | Erster Inkontinenzartikel 1A | | | Zweiter Inkontinenzartikel 1B | | |
|---|---|---|---|---|---|---|
| | Menge [g] | Flächeng ewicht^{1,2} [g/m²] | Gewichtspr ozent des pH-Regulations mittels [%] | Menge [g] | Flächengew icht^{1,2} [g/m²] | Gewichtsproz ent des pH-Regulationsmi ttels [%] |
| Mittlerer Längsabsc hnitt | 0,40 ± 0,02 | 9,00 ± 0,36 | 67,9 ± 2,8 | 0,65 ± 0,02 | 14,18 ± 0,41 | 91,9 ± 2,7 |
| Vorderer Längsabsc hnitt | 0,06 ± 0,01 | 1,79 ± 0,35 | 10,0 ± 1,7 | 0,01 ± 0,00 | 0,38 ± 0,04 | 1,8 ± 0,2 |
| Hinterer Längsabsc hnitt | 0,13 ± 0,01 | 3,32 ± 0,39 | 22,1 ± 2,2 | 0,04 ± 0,01 | 1,16 ± 0,24 | 6,3 ± 1,3 |
| Von ADL überfangen er Bereich | 0,37 ± 0,02 | 16,98 ± 0,80 | 61,1 ± 2,9 | 0,57 ± 0,02 | 26,48 ± 0,94 | 81,7 ± 2,9 |
| Kanal | | 16,98 ± 0,80 | | | 26,48 ± 0,94 | |
| insgesamt | 0,60 | | 100,0 | 0,70 | | 100,0 |
| ¹ zu verstehen als durchschnittliches Flächengewicht innerhalb des genannten Bereichs oder Längsabschnitts, ² flächenbezogene Menge | | | | | | |

**Tabelle 2: Abmessungen des ersten 1A und zweiten Inkontinenzartikels 1B**

| | Erstreckung in Längsrichtung³ [mm] | Längserstreckung in Prozent [%] der Gesamterstreckung des Absorptionskörpers⁴ |
|---|---|---|
| Absorptionskörper | 560 | 100 |
| Mittlerer Längsabschnitt | 240 | 43 |
| Vorderer Längsabschnitt | 160 | 29 |
| Hinterer Längsabschnitt | 160 | 29 |
| ³ zu verstehen als maximale Erstreckung in Längsrichtung des Absorptionskörpers; ⁴ gerundet | | |

### Testmethode pH-Wert-Messung an der Oberfläche eines Inkontinenzartikels:

Zur Durchführung einer pH-Wert-Messung an der Oberfläche eines Inkontinenzartikels, insbesondere auf einer äußeren Oberseite des Topsheets des Inkontinenzartikels, wird eine Urinersatzlösung verwendet. Die Urinersatzlösung ist eine nach ISO 11984-1 (1996) hergestellte 0,9 %ige (w/v) Lösung aus Natriumchlorid (NaCl) in destilliertem Wasser, welche mit Natriumhydroxid (NaOH) auf pH 6.8 eingestellt ist. Eine solche Lösung ist im allgemeinen Fachwissen seit langem bekannt und deren Herstellung gehört zu den Routinemethoden des Fachgebiets.

Der zu testende Inkontinenzartikel wird mit der Topsheet-Seite nach oben flach ausgebreitet, dabei gegebenenfalls vollständig aufgefaltet und gegebenenfalls auf einer Unterlage fixiert.

Der Test wird entsprechend der ISO 11984-1 (1996) bei einer Temperatur von 23 °C ± 2 °C und einer relativen Luftfeuchtigkeit von 50% ± 5% durchgeführt. Der zu testende Inkontinenzartikel und die Urinersatzflüssigkeit werden gemäß ISO 11984-1 (1996) entsprechend vorkonditioniert.

Um eine Benutzungssituation relativ realitätsnah abzubilden und die pH-Wert-Entwicklung oder die Pufferwirkung des pH-Regulationsmittels über eine längere Benutzungsdauer mit mehreren Miktionsereignissen zu verfolgen, werden wie nachfolgend beschrieben im Verlauf des Tests drei Miktionsereignisse über einen Zeitraum von fünf Stunden simuliert. Der Inkontinenzartikel wird hierbei insgesamt mit einem Flüssigkeitsvolumen beladen, das etwa 90% einer maximalen Absorptionskapazität des Inkontinenzartikels entspricht.

Die Volumina der ersten und zweiten und dritten simulierten Miktion sind daher so zu wählen, dass ein Gesamtvolumen der ersten und zweiten und dritten simulierten Miktion etwa 90% der maximalen Absorptionskapazität des Inkontinenzartikels entspricht. Dabei entspricht ein Volumen einer ersten simulierten Miktion 45% bis 50% der maximalen Absorptionskapazität des Inkontinenzartikels. Ein jeweiliges Volumen einer zweiten und einer dritten simulierten Miktion ist im Wesentlichen gleich und entspricht jeweils 20% bis 22,5% der maximalen Absorptionskapazität.

Falls die maximale Absorptionskapazität eines Inkontinenzartikels nicht bekannt ist, wird die maximale Absorptionskapazität anhand eines zweiten vergleichbaren Inkontinenzartikels, beispielsweise eines derselben Saugstärke und Größe und Produktart zuzuordnenden und gegebenenfalls derselben Packung zu entnehmenden Inkontinenzartikels wie der zu testende Inkontinenzartikel, vorab gemäß ISO 11984-1 (1996) bestimmt.

Ein Volumen der Urinersatzlösung, welches 50% der maximalen Absorptionskapazität des Inkontinenzartikels entspricht, wird innerhalb von 30 Sekunden zum Zweck der Simulierung einer ersten Miktion auf den trockenen Inkontinenzartikel gegossen und zwar derart, dass die Urinersatzlösung in einem Bereich auf den Inkontinenzartikel trifft, in welchem auch bei einem Miktionsereignis im Benutzungszustand die Miktionsflüssigkeit auf den Inkontinenzartikel treffen würde. Sollte dieser Bereich nicht zweifelsfrei feststehen, soll die Urinersatzlösung mittig auf den Absorptionskörper, insbesondere im Bereich einer Quermittelachse des Absorptionskörpers treffen. Falls der Inkontinenzartikel eine Flüssigkeitsaufnahme- und Verteilerschicht (ADL) aufweist, wird die Urinersatzlösung insbesondere mittig innerhalb eines von der ADL gebildeten Bereiches auf den Inkontinenzartikel gegossen.

Zwei Stunden nach Beginn der ersten simulierten Miktion mit dem ersten Volumen der Urinersatzlösung wird ein zweites Volumen der Urinersatzlösung, welches 20 % der maximalen Absorptionskapazität des Inkontinenzartikels entspricht, wie oben beschrieben auf den Inkontinenzartikel gegossen, um eine zweite Miktion zu simulieren.

Fünf Stunden nach Beginn der Beladung mit dem ersten Volumen der Urinersatzlösung wird ein drittes Volumen der Urinersatzlösung, welches 20 % der maximalen Absorptionskapazität des Inkontinenzartikels entspricht, wie oben beschrieben auf den Inkontinenzartikel gegossen, um eine dritte Miktion zu simulieren.

Nach jedem der drei simulierten Miktionsereignisse wird der pH-Wert an der äußeren Oberseite des Topsheets des Inkontinenzartikels 1, 3, 5, 10, 15 und 20 min nach Ende der jeweiligen simulierten Miktion gemessen. Dazu wird der pH-Wert an jeweils 4 Messpunkten gemessen, welche in Längs- und/oder Querrichtung des flach ausgebreiteten Inkontinenzartikels voneinander beabstandet sind und ein Durchschnittswert der vier gemessenen Werte gebildet. Falls eine ADL vorhanden ist, sollen die Messungen innerhalb des von der ADL gebildeten Bereiches erfolgen.

In jedem Fall sind die Messpunkte innerhalb eines Bereiches, welcher pH-Regulationsmittel aufweist, anzuordnen. Die Messpunkte sollen voneinander beabstandet sein, insbesondere zumindest nicht miteinander überlappen.

Es ist grundsätzlich darauf zu achten, dass die Messpunkte des Inkontinenzartikels nicht zu trocken sind, um verlässliche Messergebnisse zu erhalten. Bevorzugt sollen die Positionen der 4 Messpunkte bei allen Messungen beibehalten werden, jedoch ist auch möglich, die Position eines oder mehrerer Messpunkte zu variieren, beispielsweise falls ein Messpunkt zum Zeitpunkt einer späteren Messung zu trocken sein sollte. Das kann beispielsweise bei Inkontinenzartikeln mit besonders niedriger Rücknässung insbesondere 15 und/oder 20 Minuten nach der jeweiligen simulierten Miktion der Fall sein. Sollten nicht wenigstens zwei geeignete Messpunkte für eine Messung zu einem bestimmten Zeitpunkt zur Verfügung stehen, wird die Messung nur zu den jeweils anderen Zeitpunkten nach Ende der jeweiligen simulierten Miktion ausgewertet. Sollten für eine Messung nur zwei oder drei Messpunkte zur Verfügung stehen ist ein Durchschnittswert der zwei oder drei gemessenen Werte zu bilden.

Aufgrund des Routine-Charakters solcher pH-Messungen im Fachgebiet ist es dem Fachmann leicht möglich, eine Beurteilung vorzunehmen, ob ein möglicher Messpunkt zu trocken ist.

Die Messung kann grundsätzlich mit jedem Messgerät vorgenommen werden, das sich nach Einschätzung des Fachmanns für die Oberflächenmessung von pH-Werten eignet. Beispielsweise eignen sich die Elektroden der Modelle HI14142 oder HI1413 von HANNA Instruments. Das Messgerät wird vor Beginn der Messung entsprechend der Herstellerangaben kalibriert

## Patentansprüche

1. Inkontinenzartikel für die Aufnahme von Körperausscheidungen, umfassend ein zumindest bereichsweise flüssigkeitsdurchlässiges Topsheet (2), ein im Wesentlichen flüssigkeitsundurchlässiges Backsheet (3) und einen zwischen dem Topsheet (2) und dem Backsheet (3) angeordneten Absorptionskörper (4), wobei der Absorptionskörper (4) eine erste Saugkörperlage (5) umfasst, und wobei die erste Saugkörperlage (5) mindestens einen in der Längsrichtung (11) orientierten Kanal (16) aufweist, wobei der Kanal (16) eine sich durch die erste Saugkörperlage (5) in Dickenrichtung (21) hindurcherstreckende Ausnehmung umfasst, **dadurch gekennzeichnet dass** ein pH-Regulationsmittel (7) in den Kanal (16) eingebracht ist.

2. Inkontinenzartikel nach Anspruch 1 **dadurch gekennzeichnet, dass** die erste Saugkörperlage (5) einen ersten Bereich (22) aufweist, und wobei die erste Saugkörperlage (5) in dem ersten Bereich (22) SAP (20) aufweist.

3. Inkontinenzartikel nach Anspruch 2 **dadurch gekennzeichnet dass** der erste Bereich (22) an den Kanal (16) angrenzt.

4. Inkontinenzartikel nach einem oder mehreren der vorhergehenden Ansprüche **dadurch gekennzeichnet dass** der Absorptionskörper (4) eine zweite Saugkörperlage (6) aufweist, wobei die zweite Saugkörperlage (6) unterhalb der ersten Saugkörperlage (5), also zwischen der ersten Saugkörperlage (5) und dem Backsheet (3) angeordnet ist.

5. Inkontinenzartikel nach Anspruch 4 **dadurch gekennzeichnet dass** die zweite Saugkörperlage (6) weniger als 20 Gewichtsprozent, 15 Gewichtsprozent, 10 Gewichtsprozent, 5 Gewichtsprozent SAP (20) aufweist, insbesondere frei von SAP (20) ist.

6. Inkontinenzartikel nach einem oder mehreren der vorhergehenden Ansprüche **dadurch gekennzeichnet dass** der Kanal (16) auf einer Längsmittelachse des Inkontinenzartikels angeordnet ist.

7. Inkontinenzartikel nach einem oder mehreren der vorhergehenden Ansprüche **dadurch gekennzeichnet dass** das pH-Regulationsmittel (7) partikelförmig ausgebildet ist.

8. Inkontinenzartikel nach Anspruch 7 **dadurch gekennzeichnet dass** mindestens 50 Gewichtsprozent des pH-Regulationsmittels (7) eine Partikelgröße von 10 bis 2000 µm, insbesondere von 50 bis 1200 µm, weiter insbesondere von 80 bis 800 µm aufweist.

9. Inkontinenzartikel nach einem oder mehreren der vorhergehenden Ansprüche **dadurch gekennzeichnet dass** die flächenbezogene Menge an pH-Regulationsmittel (7) in dem Kanal (16) 5 bis 100 g/m², insbesondere 10 bis 50 g/m², weiter insbesondere 15 bis 30 g/m² beträgt.

10. Inkontinenzartikel nach einem oder mehreren der vorhergehenden Ansprüche **dadurch gekennzeichnet dass** das pH-Regulationsmittel (7) Trinatriumcitrat, Mononatriumcitrat oder Dinatriumcitrat aufweist.

11. Inkontinenzartikel nach Anspruch 10, wobei das pH-Regulationsmittel (7) aus Mononatriumcitrat oder Dinatriumcitrat besteht.

12. Inkontinenzartikel nach einem oder mehreren der Ansprüche 2 bis 11 **dadurch gekennzeichnet dass** die erste Saugkörperlage (5) in dem ersten Bereich (22) zumindest eine Teilmenge des pH-Regulationsmittels (7) aufweist.

13. Inkontinenzartikel nach einem oder mehreren der vorhergehenden Ansprüche **dadurch gekennzeichnet dass** das pH-Regulationsmittel (7) in der ersten Saugkörperlage (5) eingebracht ist, wobei das pH-Regulationsmittel (7) in der ersten Saugkörperlage (5) ungleichförmig eingebracht ist, insbesondere derart, dass die flächenbezogene Menge des pH-Regulationsmittels (7) in einem mittleren Längsabschnitt des Absorptionskörpers (4) in g/m² größer ist als in einem vorderen und/oder einem hinteren Längsabschnitt des Absorptionskörpers (4).

14. Inkontinenzartikel nach Anspruch 13 **dadurch gekennzeichnet dass** 5,00-30,00 g/m², insbesondere 6,00-25,00 g/m², weiter insbesondere 7,00-20,00 g/m², weiter insbesondere 8,00-15,00 g/m² pH-Regulationsmittel (7) in dem mittleren Längsabschnitt des Absorptionskörpers (4) angeordnet ist und wobei in dem vorderen und/oder hinteren Längsabschnitt des Absorptionskörpers (4) jeweils 0,00-10,00 g/m², insbesondere 0,10-9,00 g/m², weiter insbesondere 0,20-7,00 g/m², weiter insbesondere 0,30-5,00 g/m² pH-Regulationsmittel (7) angeordnet ist.

15. Inkontinenzartikel nach einem oder mehreren der Ansprüche 2 bis 14 **dadurch gekennzeichnet dass** das SAP (20) in der ersten Saugkörperlage (5) derart eingebracht ist, dass die Konzentration des SAP (20) in Gewichtsprozent in dem mittleren Längsabschnitt des ersten Bereichs (22) der ersten Saugkörperlage (5) gleich groß ist wie in dem vorderen und/oder dem hinteren Längsabschnitt der ersten Saugkörperlage (5).

16. Inkontinenzartikel nach einem oder mehreren der Ansprüche 13 bis 15 **dadurch gekennzeichnet dass** das pH-Regulationsmittel (7) in der ersten Saugkörperlage (5) derart eingebracht ist, dass die Konzentration des pH-Regulationsmittels (7) in Gewichtsprozent in dem mittleren Längsabschnitt des Absorptionskörpers (4) größer ist als dem vorderen und/oder dem hinteren Längsabschnitt des Absorptionskörpers (4).

17. Inkontinenzartikel nach einem oder mehreren der vorhergehenden Ansprüche **dadurch gekennzeichnet dass** der Inkontinenzartikel mindestens eine Flüssigkeitsaufnahme- und Verteilerschicht (ADL) (15) aufweist, welche zwischen dem Topsheet (2) und der ersten Saugkörperlage (5) angeordnet ist.

18. Inkontinenzartikel nach Anspruch 17 **dadurch gekennzeichnet dass** die ADL (15) den Kanal (16) bereichsweise, insbesondere vollständig überfängt.

19. Inkontinenzartikel nach einem oder mehreren der vorhergehenden Ansprüche **dadurch gekennzeichnet dass** der Inkontinenzartikel über mindestens zwei, insbesondere mindestens drei Miktionsereignisse hinweg, einen hautfreundlichen pH-Wert aufrechterhält.

20. Inkontinenzartikel nach Anspruch 19 **dadurch gekennzeichnet dass** der pH-Wert auf einer der Haut zugewandten Oberseite des Inkontinenzartikels einen Wert von 4,0-6,5, insbesondere 4,2-6,2, weiter insbesondere 4,3-6,0, weiter insbesondere 4,5-5,8, weiter insbesondere 4,7-5,7 beträgt.

21. Inkontinenzartikel nach einem oder mehreren der Ansprüche 2 bis 20 **dadurch gekennzeichnet dass** der Kanal (16) der ersten Saugkörperlage (5) im Wesentlichen frei von SAP (20) ist.

22. Inkontinenzartikel nach einem oder mehreren der Ansprüche 4 bis 21 **dadurch gekennzeichnet dass** die zweite Saugkörperlage (6) weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels (7) aufweist, insbesondere frei von pH-Regulationsmittel (7) ist.

## Claims

1. Incontinence article for accommodating bodily excretions, comprising an at least regionally liquid-permeable topsheet (2), a substantially liquid-impermeable backsheet (3), and an absorption body (4) arranged between the topsheet (2) and the backsheet (3), the absorption body (4) comprising a first adsorbent ply (5) and the first absorbent ply (5) having at least one longitudinally (11) oriented channel (16), wherein the channel (16) comprises a cutout extending in thickness direction (21) through the first absorbent ply (5), **characterized in that** there is a pH regulator (7) introduced in the channel (16).

2. Incontinence article according to Claim 1, **characterized in that** the first absorbent ply (5) has a first region (22) and wherein the first absorbent ply (5) comprises SAP (20) in the first region (22).

3. Incontinence article according to Claim 2, **characterized in that** the first region (22) borders the channel (16).

4. Incontinence article according to one or more of the preceding claims, **characterized in that** the absorption body (4) has a second absorbent ply (6), the second absorbent ply (6) being arranged beneath the first absorbent ply (5), i.e., between the first absorbent ply (5) and the backsheet (3).

5. Incontinence article according to Claim 4, **characterized in that** the second absorbent ply (6) comprises less than 20 percent by weight, 15 percent by weight, 10 percent by weight, 5 percent by weight of SAP (20), and more particularly is free of SAP (20).

6. Incontinence article according to one or more of the preceding claims, **characterized in that** the channel (16) is arranged on a longitudinal central axis of the incontinence article.

7. Incontinence article according to one or more of the preceding claims, **characterized in that** the pH regulator (7) is particulate.

8. Incontinence article according to Claim 7, **characterized in that** at least 50 percent by weight of the pH regulator (7) has a particle size of 10 to 2000 µm, more particularly of 50 to 1200 µm, more particularly still of 80 to 800 µm.

9. Incontinence article according to one or more of the preceding claims, **characterized in that** the amount of pH regulator (7) per unit area in the channel (16) is 5 to 100 g/m², more particularly 10 to 50 g/m², more particularly still 15 to 30 g/m².

10. Incontinence article according to one or more of the preceding claims, **characterized in that** the pH regulator (7) comprises trisodium citrate, monosodium citrate or disodium citrate.

11. Incontinence article according to Claim 10, wherein the pH regulator (7) consists of monosodium citrate or disodium citrate.

12. Incontinence article according to one or more of Claims 2 to 11, **characterized in that** the first absorbent ply (5) in the first region (22) comprises at least a portion of the pH regulator (7).

13. Incontinence article according to one or more of the preceding claims, **characterized in that** the pH regulator (7) is introduced in the first absorbent ply (5), the pH regulator (7) being introduced heterogeneously in the first absorbent ply (5), more particularly in such a way that the amount of the pH regulator (7) per unit area in a middle longitudinal section of the absorption body (4) in g/m² is greater than in a front and/or a back longitudinal section of the absorption body (4).

14. Incontinence article according to Claim 13, **characterized in that** 5.00-30.00 g/m², more particularly 6.00-25.00 g/m², more particularly still 7.00-20.00 g/m², more particularly still 8.00-15.00 g/m² of pH regulator (7) are arranged in the middle longitudinal section of the absorption body (4) and wherein in the front and/or rear longitudinal section of the absorption body (4) there are arranged in each case 0.00-10.00 g/m², more particularly 0.10-9.00 g/m², more particularly still 0.20-7.00 g/m², more particularly still 0.30-5.00 g/m² of pH regulator (7).

15. Incontinence article according to one or more of Claims 2 to 14, **characterized in that** the SAP (20) is introduced in the first absorbent ply (5) in such a way that the concentration of the SAP (20) in percent by weight in the middle longitudinal section of the first region (22) of the first absorbent ply (5) is the same as in the front and/or the back longitudinal section of the first absorbent ply (5).

16. Incontinence article according to one or more of Claims 13 to 15, **characterized in that** the pH regulator (7) is introduced in the first absorbent ply (5) in such a way that the concentration of the pH regulator (7) in percent by weight in the middle longitudinal section of the absorption body (4) is greater than in the front and/or the back longitudinal section of the absorption body (4).

17. Incontinence article according to one or more of the preceding claims, **characterized in that** the incontinence article has at least one liquid acquisition distribution layer (ADL) (15) which is arranged between the topsheet (2) and the first absorbent ply (5).

18. Incontinence article according to Claim 17, **characterized in that** the ADL (15) overlies the channel (16) regionally, more particularly completely.

19. Incontinence article according to one or more of the preceding claims, **characterized in that** the incontinence article maintains a skin-friendly pH over at least two, in particular at least three, micturition events.

20. Incontinence article according to Claim 19, **characterized in that** the pH on a skin-facing top side of the incontinence article is a value of 4.0-6.5, more particularly 4.2-6.2, more particularly still 4.3-6.0, more particularly still 4.5-5.8, more particularly still 4.7-5.7.

21. Incontinence article according to one or more of Claims 2 to 20, **characterized in that** the channel (16) of the first absorbent ply (5) is substantially free of SAP (20).

22. Incontinence article according to one or more of Claims 4 to 21, **characterized in that** the second absorbent ply (6) comprises less than 20 percent by weight, more particularly less than 15 percent by weight, more particularly still less than 10 percent by weight, more particularly still less than 5 percent by weight of the pH regulator (7) and more particularly is free of pH regulator (7).

## Revendications

1. Article pour incontinence destiné à recueillir des excrétions corporelles, comprenant une feuille supérieure (2) perméable aux liquides au moins par zones, une feuille arrière (3) sensiblement imperméable aux liquides et un corps absorbant (4) disposé entre la feuille supérieure (2) et la feuille arrière (3), le corps absorbant (4) comprenant une première couche absorbante (5) et la première couche absorbante (5) présentant au moins un canal (16) orienté dans la direction longitudinale (11), le canal (16) comprenant un évidement s'étendant à travers la première couche absorbante (5) dans la direction de l'épaisseur (21), **caractérisé en ce qu'**un régulateur de pH (7) est introduit dans le canal (16).

2. Article pour incontinence selon la revendication 1, **caractérisé en ce que** la première couche absorbante (5) présente une première zone (22) et la première couche absorbante (5) présentant un SAP (polymère superabsorbant) (20) dans la première zone (22).

3. Article pour incontinence selon la revendication 2, **caractérisé en ce que** la première zone (22) est adjacente au canal (16).

4. Article pour incontinence selon l'une ou plusieurs des revendications précédentes, **caractérisé en ce que** le corps absorbant (4) présente une seconde couche absorbante (6), la seconde couche absorbante (6) étant agencée sous la première couche absorbante (5), c'est-à-dire entre la première couche absorbante (5) et la feuille arrière (3).

5. Article pour incontinence selon la revendication 4, **caractérisé en ce que** la seconde couche absorbante (6) présente moins de 20 % en poids, moins de 15 % en poids, moins de 10 % en poids, moins de 5 % en poids de SAP (20) et est en particulier exempte de SAP (20).

6. Article pour incontinence selon l'une ou plusieurs des revendications précédentes, **caractérisé en ce que** le canal (16) est agencé sur un axe central longitudinal de l'article pour incontinence.

7. Article pour incontinence selon l'une ou plusieurs des revendications précédentes, **caractérisé en ce que** le régulateur de pH (7) est réalisé sous forme de particules.

8. Article pour incontinence selon la revendication 7, **caractérisé en ce qu'**au moins 50 % en poids du régulateur de pH (7) présentent une grosseur de particule de 10 à 2000 µm, en particulier de 50 à 1200 µm, plus particulièrement de 80 à 800 µm.

9. Article pour incontinence selon l'une ou plusieurs des revendications précédentes, **caractérisé en ce que** la quantité, rapportée à la surface, de régulateur de pH (7) dans le canal (16) est de 5 à 100 g/m², en particulier de 10 à 50 g/m², plus particulièrement de 15 à 30 g/m².

10. Article pour incontinence selon l'une ou plusieurs des revendications précédentes, **caractérisé en ce que** le régulateur de pH (7) présente du citrate trisodique, du citrate monosodique ou du citrate disodique.

11. Article pour incontinence selon la revendication 10, le régulateur de pH (7) étant constitué de citrate monosodique ou de citrate disodique.

12. Article pour incontinence selon l'une ou plusieurs des revendications 2 à 11, **caractérisé en ce que** la première couche absorbante (5) présente, dans la première zone (22), au moins une quantité partielle de régulateur de pH (7).

13. Article pour incontinence selon l'une ou plusieurs des revendications précédentes, **caractérisé en ce que** le régulateur de pH (7) est incorporé dans la première couche absorbante (5), le régulateur de pH (7) étant incorporé de manière non uniforme dans la première couche absorbante (5), en particulier de manière à ce que la quantité, rapportée à la surface, de régulateur de pH (7) dans une section longitudinale centrale du corps absorbant (4) en g/m² soit supérieure à celle dans une section longitudinale avant et/ou arrière du corps absorbant (4).

14. Article pour incontinence selon la revendication 13, **caractérisé en ce que** 5,00-30,00 g/m², en particulier 6,00-25,00 g/m², plus particulièrement 7,00-20,00 g/m², plus particulièrement 8,00-15,00 g/m² de régulateur de pH (7) sont agencés dans la section longitudinale centrale du corps absorbant (4) et à chaque fois 0,00-10,00 g/m², en particulier 0,10-9,00 g/m², plus particulièrement 0,20-7,00 g/m², plus particulièrement 0,30-5,00 g/m² de régulateur de pH (7) sont agencés dans la section longitudinale avant et/ou arrière du corps absorbant (4).

15. Article pour incontinence selon l'une ou plusieurs des revendications 2 à 14, **caractérisé en ce que** le SAP (20) est incorporé dans la première couche absorbante (5) de manière à ce que la concentration en SAP (20), en pourcentage en poids, dans la section longitudinale centrale de la première zone (22) de la première couche absorbante (5) soit égale à celle dans la section longitudinale avant et/ou arrière de la première couche absorbante (5).

16. Article pour incontinence selon l'une ou plusieurs des revendications 13 à 15, **caractérisé en ce que** le régulateur de pH (7) est incorporé dans la première couche absorbante (5) de manière à ce que la concentration en régulateur de pH (7), en pourcentage en poids, dans la section longitudinale centrale du corps absorbant (4) soit supérieure à celle dans la section longitudinale avant et/ou arrière du corps absorbant (4).

17. Article pour incontinence selon l'une ou plusieurs des revendications précédentes, **caractérisé en ce que** l'article pour incontinence présente au moins une couche absorbant et répartissant le liquide (ADL) (15), qui est agencée entre la feuille supérieure (2) et la première couche absorbante (5).

18. Article pour incontinence selon la revendication 17, **caractérisé en ce que** l'ADL (15) recouvre partiellement le canal (16), en particulier complètement.

19. Article pour incontinence selon l'une ou plusieurs des revendications précédentes, **caractérisé en ce que** l'article pour incontinence conserve une valeur de pH respectueuse de la peau sur au moins deux, en particulier sur au moins trois événements mictionnels.

20. Article pour incontinence selon la revendication 19, **caractérisé en ce que** la valeur de pH sur une face supérieure orientée vers la peau de l'article pour incontinence présente une valeur de 4,0-6,5, en particulier de 4,2-6,2, plus particulièrement de 4,3-6,0, plus particulièrement de 4,5-5,8, plus particulièrement de 4,7-5,7.

21. Article pour incontinence selon l'une ou plusieurs des revendications 2 à 20, **caractérisé en ce que** le canal (16) de la première couche absorbante (5) est sensiblement exempt de SAP (20).

22. Article pour incontinence selon l'une ou plusieurs des revendications 4 à 21, **caractérisé en ce que** la seconde couche absorbante (6) présente moins de 20 % en poids, en particulier moins de 15 % en poids, plus particulièrement moins de 10 % en poids, plus particulièrement moins de 5 % en poids, de régulateur de pH (7) et **en ce qu'**elle est en particulier exempte de régulateur de pH (7).
